# EUROPEAN PATENT APPLICATION

(11) **EP 4 261 279 A1**
(43) Date of publication of application: **18.10.2023**
(21) Application number: 21903894.0
(22) Date of filing: 10.12.2021
(51) Int. Cl.: C12N 9/00, A61K 38/53, A61P 37/04, A61P 35/00, A61K 39/00

(54) **NOVEL FRAGMENTED CRS PEPTIDE EXHIBITING IMMUNE ENHANCEMENT ACTIVITY, AND USE THEREOF**

(30) Priority: 10.12.2020 KR 20200172565
(71) Applicant: Zymedi Co., Ltd., Incheon 21983 (KR)
(72) Inventor: KIM, Sung Hoon, Incheon 21983 (KR); KIM, Sang Bum, Incheon 21983 (KR); CHO, Seongmin, Incheon 21983 (KR); SONG, Ee Chan, Incheon 21983 (KR)
(74) Representative: Hoefer & Partner Patentanwälte mbB
(86) International application number: PCT/KR2021/018751
(87) International publication number: WO 2022/124849

(57) **Abstract**

The present invention relates to a novel fragmented CRS peptide exhibiting immune enhancement activity, and a use thereof, and, more specifically, to a novel peptide consisting of an amino acid sequence of SEQ ID NO: 2, and a use thereof as a vaccine adjuvant and a cancer therapeutic agent. A peptide disclosed in the present invention is a CRS fragment disclosed for the first time in the present specification and exhibits an anti-cancer activity and immune enhancement activity.

## Description

### THECHNICAL FIELD

This application claims priority to Korean Patent Application No. 10-2020-0172565 filed on December 10, 2020, and the entire specifications of which are incorporated herein by reference in their entireties.

The present invention relates to a novel fragmented CRS peptide exhibiting immune enhancement activity and use thereof, more specifically, it relates to a novel peptide consisting of the amino acid sequence of SEQ ID NO: 2, its vaccine adjuvant, and its use as a cancer treatment.

### BACKGROUND OF THE INVENTION

Aminoacyl-tRNA synthetases (ARS or AARS), which catalyze the aminoacylation of tRNA molecules, are essential for decoding genetic information during the translation process. Each of the eukaryotic tRNA synthetases consists of a core enzyme (closely related to the prokaryotic counterpart of tRNA synthetase) and additional domains (It is added to the amino terminal or carboxy terminal of the core enzyme). Thus, there are significant differences in the composition of these enzymes between eukaryotes and prokaryotes. For example, human tyrosyl-tRNA synthetase (TyrRS) has a carboxy terminal domain that is absent from prokaryotic and lower eukaryotic TyrRS molecules.

Several aminoacyl-tRNA synthetases have recently been demonstrated to have non-canonical functions independent of their involvement in the translational process. That is, in some fragments of the ARS protein, it has been identified that they exhibit extracellular signaling activity that regulates other types of pathways beyond protein translation and possess unexpected activities not related to aminoacylation. Such unexpected activity may sometimes be a therapeutically usable activity for a specific disease or the like, but in some cases, it may be an activity that has a high risk of functioning to cause a disease state in humans. For example, it has been found that lysyl-tRNA synthetase (KRS) has an activity that promotes cancer metastasis (Korean Registered Patent No. 10-1453141). In addition, mini-tyrosyl tRNA synthetase (mini-TRS, corresponding to amino acid residues 1-364), the N-terminal domain of TRS, which is cleaved by polymorphonuclear cell elastase and plasmin, exhibits non-canonical biological activity not found in full-length proteins, in vitro, mini-TRS was shown to stimulate endothelial cell proliferation and migration and has pro-angiogenic activity in mouse matrigel assay. The function of promoting angiogenesis is generally closely related to cancer metastasis.

As such, this unexpected activity is not observed in natural full-length protein sequences (or show insignificant effects at native full-length protein levels), there are cases in which a specific activity is markedly exhibited when some regions are isolated, and there are also cases in which the effect possesses properties unsuitable for therapeutic use. In order to overcome the difficulties due to this unpredictability and utilize the therapeutic potential of these ARS family proteins, various efforts are required to identify biologically relevant forms of other aminoacyl-tRNA synthetase proteins.

On the other hand, the pharmaceutical industry is changing from natural products or chemically synthesized drugs in the past to the development of protein or peptide drugs, among the global pharmaceutical markets, the market for protein or peptide drugs expanded from $43.7 billion in 2006 to $88.5 billion in 2011, the share of the domestic protein drug market in the global market is expected to increase from 3% in 2006 to 7% in 2021. Protein or peptide drugs are evaluated as an innovative field of medicine because they have fewer side effects and faster drug effects than synthetic drugs.

Currently, the importance of biopharmaceuticals in the pipeline of major pharmaceutical companies is gradually increasing, but major technical problems exist until specific biopharmaceuticals such as peptides are released. For example, the low delivery rate of peptide drugs to the target site and the synthesis of long-chain peptides are acting as obstacles to commercialization. It is known that the key to success as a peptide drug is to discover a short sequence that shows activity, that is, to select a minimal motif with excellent physiological activity from a full-length protein. If the peptide length is long, it is known that the synthesis cost is high, the preparation is not easy, and there is a problem in human body absorption.

In this regard, Korean Patent Application No. 10-2019-0026490 discloses a fragment of CRS protein (106-228aa) and use thereof, The CRS fragment peptide disclosed in the literature not only forms a multimer, but also has limitations in being developed as a drug because the peptide is degraded if the affinity tag attached to the N-terminus and C-terminus is removed.

### DETAILED DESCRIPTION OF THE INVENTION

### TECHNICAL PROBLEM

The present inventors have found that a fragment peptide of cysteineyl-tRNA synthetase (hereinafter referred to as 'CRS') has anticancer activity and immune enhancing activity in a previous study, but it was confirmed that it is difficult to develop it as a drug due to the above-mentioned limitations. Accordingly, as a result of intensive research to develop a novel fragmented CRS peptide capable of overcoming the above-mentioned limitations, the present inventors have found and completed the present invention that the novel fragmented CRS peptide consisting of the amino acid sequence of SEQ ID NO: 2 maintains its form as a monomer and does not degrade without an affinity tag while exhibiting anticancer activity and immune function enhancing activity to an equivalent degree compared to the conventional CRS fragment peptide described above.

Accordingly, an object of the present invention is to provide a peptide consisting of the amino acid sequence of SEQ ID NO: 2 or an amino acid sequence showing 95% or more sequence homology thereto.

Another object of the present invention is to provide a polynucleotide comprising a nucleotide sequence encoding the peptide.

Another object of the present invention is to provide a vector comprising the polynucleotide.

Another object of the present invention is to provide a host cell transformed with the vector.

Another object of the present invention is to provide a vaccine adjuvant comprising at least one selected from the group consisting of the following (i) to (iv):
(i) the peptide,
(ii) a polynucleotide encoding (i) the peptide;
(iii) a vector comprising (ii) the polynucleotide, and
(iv) a host cell transformed by (iii) the vector.

Another object of the present invention is to provide a vaccine composition comprising the vaccine adjuvant and antigen.

Another object of the present invention is to provide a pharmaceutical composition for preventing or treating cancer comprising at least one selected from the group consisting of the following (i) to (iv).

In addition, another object of the present invention is to provide a pharmaceutical composition for preventing or treating cancer comprising at least one selected from the group consisting of the following (i) to (iv).

In addition, another object of the present invention is to provide a pharmaceutical composition for preventing or treating cancer essentially consisting of at least one selected from the group consisting of the following (i) to (iv):
(i) the peptide,
(ii) a polynucleotide encoding (i) the peptide;
(iii) a vector comprising (ii) the polynucleotide, and
(iv) a host cell transformed by (iii) the vector.

Another object of the present invention is to provide use of the at least one selected from the group consisting of the following (i) to (iv) for preparation of an agent for the treatment of cancer:
(i) the peptide,
(ii) a polynucleotide encoding (i) the peptide;
(iii) a vector comprising (ii) the polynucleotide, and
(iv) a host cell transformed by (iii) the vector.

Another object of the present invention is to provide a method for treating cancer comprising administering to a subject in need thereof an effective amount of a composition comprising at least one selected from the group consisting of the following (i) to (iv):
(i) the peptide,
(ii) a polynucleotide encoding (i) the peptide;
(iii) a vector comprising (ii) the polynucleotide, and
(iv) a host cell transformed by (iii) the vector.

### TECHNICAL SOLUTION

In order to achieve the above object of the present invention, the present invention provides a peptide consisting of the amino acid sequence of SEQ ID NO: 2 or an amino acid sequence showing 95% or more sequence homology thereto.

In order to achieve the above object of the present invention, the present invention provides a polynucleotide comprising a nucleotide sequence encoding the peptide.

In order to achieve the above object of the present invention, the present invention provides a vector comprising the polynucleotide.

In order to achieve the above object of the present invention, the present invention provides a host cell transformed with the vector.

In order to achieve the above object of the present invention, the present invention provides a vaccine adjuvant comprising at least one selected from the group consisting of the following (i) to (iv).
(i) the peptide,
(ii) a polynucleotide encoding (i) the peptide;
(iii) a vector comprising (ii) the polynucleotide, and
(iv) a host cell transformed by (iii) the vector.

In order to achieve the above object of the present invention, the present invention provides a vaccine composition comprising the vaccine adjuvant and an antigen.

In order to achieve another object of the present invention, the present invention provides a pharmaceutical composition for preventing or treating cancer comprising at least one selected from the group consisting of the following (i) to (iv).

In addition, the present invention provides a pharmaceutical composition for preventing or treating cancer consisting of at least one selected from the group consisting of the following (i) to (iv).

In addition, the present invention provides a pharmaceutical composition for preventing or treating cancer essentially consisting of at least one selected from the group consisting of the following (i) to (iv).
(i) the peptide,
(ii) a polynucleotide encoding (i) the peptide;
(iii) a vector comprising (ii) the polynucleotide, and
(iv) a host cell transformed by (iii) the vector.

In order to achieve another object of the present invention, the present invention provides use of the at least one selected from the group consisting of the following (i) to (iv) for preparation of an agent for the treatment of cancer:
(i) the peptide,
(ii) a polynucleotide encoding (i) the peptide;
(iii) a vector comprising (ii) the polynucleotide, and
(iv) a host cell transformed by (iii) the vector.

In order to achieve another object of the present invention, the present invention provides a method for treating cancer comprising administering to a subject in need thereof an effective amount of a composition comprising at least one selected from the group consisting of the following (i) to (iv):
(i) the peptide,
(ii) a polynucleotide encoding (i) the peptide;
(iii) a vector comprising (ii) the polynucleotide, and
(iv) a host cell transformed by (iii) the vector.

Hereinafter, the present invention will be described in detail.

Most of the examples of the present invention are described below (literature) for the purpose of explanation, using conventional methods of molecular biology and recombinant DNA technology within the technical field to which the present invention belongs, unless specifically indicated to the contrary. These techniques are detailed in the literature: Sambrook, et al., Molecular Cloning: A Laboratory Manual (2nd Edition, 1989); Maniatis et al., Molecular Cloning: A Laboratory Manual (1982); DNA Cloning: A Practical Approach, vol. I & II (D. Glover, ed.); Oligonucleotide Synthesis (N. Gait, ed., 1984); NucleicAcid Hybridization (B. Hames & S. Higgins, eds., 1985); Transcription and Translation (B. Hames & S. Higgins, eds., 1984); Animal Cell Culture (R. Freshney, ed., 1986); A Practical Guide to Molecular Cloning (B. Perbal, ed., 1984).

All publications, patents and patent applications cited herein are hereby incorporated by reference in their entirety.

Throughout the disclosure herein, various aspects or conditions relating to the present invention may be suggested in a range format. In this specification, the description of a range value is meant to include a corresponding boundary value, that is, to include all values from the lower limit value to the upper limit value unless otherwise specified. It should be understood that the description of range formats is merely for convenience and brevity and should not be construed as an inflexible limitation to the scope of the present invention. Accordingly, statements of ranges should be considered to have specifically disclosed all possible subranges as well as individual numerical values within the range. For example, the description of a range, such as 7 to 170, can include individual values within the range, such as 10 to 127, 23 to 35, 80 to 100, 50 to 169 etc., as well as 9, 27, 35, 101, and 155 should be considered as specifically disclosed. This applies regardless of the width of the range.

The term 'comprising' herein is used in the same meaning as 'including' or 'characterized by', and does not exclude additional ingredients or steps of the method which are not specifically mentioned in the composition or the method according to the present invention. The term 'consisting of' means excluding additional elements, steps or ingredients, etc., unless otherwise described. The term 'essentially consisting of' means including substances or steps which do not substantially affect basic properties thereof in addition to the described substances or steps within the range of the composition or the method.

As used herein, the terms 'peptide' and 'protein' are used according to their usual (conventional) meaning, that is, they refer to an amino acid sequence. A peptide is not limited to a specific length, but in the context of the present invention generally refers to a fragment of a full length protein, and it may include post-translational modifications, such as glycosylation, acetylation, phosphorylation, etc., and other modifications (Naturally Occurring modifications and Non-Naturally Occurring modifications) known in the art, and may be referred to as a 'polypeptide'. The peptides and proteins of the present invention can be prepared using any of a variety of known recombinant and/or synthetic techniques, illustrative examples of which are further described below.

The present invention stems from the discovery that CRS and CRS-derived peptides possess therapeutically relevant non-canonical biological activities.

In the present specification, 'non-canonical activity' generally refers to an activity possessed by the CRS peptide of the present invention other than adding cysteine to a tRNA molecule. As detailed herein, in a specific embodiment, the non-canonical biological activity exhibited by the CRS fragment of the present invention may be selected from the group consisting of, but not limited to, anticancer activity, innate immune activation, and adaptive immune activation.

It should be understood that the scope of the present invention includes not only CRS fragment peptides having at least one non-canonical biological activity, but also variants substantially maintaining the non-canonical activity.

Specifically, the present inventors have found that the novel fragmented CRS peptide activates innate immunity and adaptive immunity to suppress tumor growth and is structurally very stable. The region of the fragmented CRS peptide is disclosed for the first time in the present invention.

Accordingly, the present invention provides a peptide consisting of the amino acid sequence of SEQ ID NO: 2 or an amino acid sequence showing 95% or more sequence homology thereto.

In the present invention, the peptide of SEQ ID NO: 2 is a truncated form of the CRS protein, and characterized in that the peptide consists of the 99th to 200th amino acids of the sequence of the CRS full-length protein consisting of the amino acid sequence of SEQ ID NO: 1, the 182nd cysteine is substituted with serine.

The peptides of the present invention can be prepared using available techniques known in the art. For example, it can be prepared using any of a variety of proteolytic enzymes. Exemplary proteases include, for example, achromopeptidase, aminopeptidase, ancrod, angiotensin converting enzyme, bromelain, calpain, calpain I, calpain II, carboxypeptidase A, carboxypeptidase B, carboxypeptidase G, carboxypeptidase P, carboxypeptidase W, carboxypeptidase Y, caspase 1, caspase 2, caspase 3, caspase 4, caspase 5, caspase 6, caspase 7, caspase 8, caspase 9, Caspase 10, Caspase 11, caspase 12, caspase 13, cathepsin B, cathepsin C, cathepsin D, cathepsin E, cathepsin G, cathepsin H, cathepsin L, chymopapain, chymase , chymotrypsin, clostripain, collagenase, complement C1r, complement C1s, complement factor D, complement factor I, cucumisin, dipeptidyl peptidase IV, leukocyte elastase, pancreatic elastase, endoproteinase Arg-C, endoproteinase Asp-N, endoproteinase Glu-C, endoproteinase Lys-C, enterokinase factor Xa, ficin, furin, granzyme A, granzyme B, HIV protease, IGase, kallikrein tissue, leucine aminopeptidase (general), leucine aminopeptidase (cytosol), leucine aminopeptidase (microsomal), matrix metalloprotease, methionine aminopeptidase, neutrase, papain, pepsin, plasmin, prolidase, pronase E, prostate specific antigen, protease alkalophilic from *Streptomyces griseus,* protease from *Aspergillus,* protease from *Aspergillus saitoi* , protease from *Aspergillus sojae,* protease B. licheniformis (alkaline or alcalase), protease from *Bacillus polymyxa,* protease from *Bacillus* sp., protease from *Rhizopus* sp., protease S, proteasomes, proteinase from *Aspergillus oryzae,* proteinase 3, proteinase A, proteinase K, protein C, pyroglutamate aminopeptidase, rennin, streptokinase, subtilisin, thermolysin, thrombin, tissue plasminogen activator, trypsin, tryptase and urokinase and the like. A person skilled in the art can easily determine which proteolytic enzyme is appropriate, taking into account the chemical specificity of the fragment to be produced.

Polypeptides described herein can be produced by any suitable procedure known to those skilled in the art, such as recombinant techniques. In addition to recombinant production methods, the polypeptides of the present invention can be prepared by direct peptide synthesis using solid phase techniques.

The solid-phase peptide synthesis (SPPS) method can initiate synthesis by attaching functional units called linkers to small porous beads and inducing them to connect peptide chains. Unlike the liquid phase method, the peptide is covalently bound to the beads and prevents them from being separated by the filtration process until they are cleaved by a specific reactant such as trifluoroacetic acid (TFA). The synthesis is performed by repeating the cycle (deprotection-wash-coupling-wash) of protection process, deprotection process, and a coupling process in which the re-exposed amine group combines with a new amino acid in which the N-terminal amine of the peptide attached to the solid phase and the N-protected amino acid unit are combined. The SPPS method can be performed using microwave technology together, and microwave technology can shorten the time required for coupling and deprotection of each cycle by applying heat in the peptide synthesis process. The thermal energy may prevent aggregation of the expanding peptide chain from forming a folding aggregate and promote chemical bonding.

In addition, the peptide of the present invention can be prepared by a liquid phase peptide synthesis method, and the specific method thereof is referred to the following documents: US Patent No. 5,516,891. In addition, the peptide of the present invention can be synthesized by various methods such as a method of mixing the solid phase synthesis method and the liquid phase synthesis method, and the preparation method is not limited to the means described herein.

Protein synthesis can be performed using manual techniques or by automation. Automated synthesis can be accomplished using, for example, an Applied Biosystems 431A Peptide Synthesizer (Perkin Elmer). Alternatively, various fragments can be chemically synthesized separately and combined using chemical methods to produce the target molecule.

The peptide provided in the present invention includes a variant having 95% or more sequence homology with the peptide of SEQ ID NO: 2. The variant refers to an active variant of the peptide, which means that the active variant retains at least one desired non-canonical activity (e.g., anticancer activity and immune enhancing activity) from the peptide from which it is derived. As an example of the variant, whether it occurs (generates), naturally or non-naturally, it may be a splice variant, and the splice variant retains at least one non-canonical activity, for example described herein. As another example, the variant, whether naturally or non-naturally occurring (generating), contains one or more point mutations to the peptide sequence, the variant peptide retains, for example, at least one non-canonical activity described herein. That is, in the present invention, the variant (or the term 'active variant') is understood as a functional equivalent of the peptide of SEQ ID NO: 2.

More specifically, the variant is characterized in that it is a functional equivalent having at least, 95% or more, 96% or more, 97% or more, 98% or more, or 99% or more sequence homology to the aforementioned peptide sequence along its length direction.

In one aspect of the present invention, the peptide may be characterized in that it consists of the amino acid sequence of SEQ ID NO: 2 or SEQ ID NO: 3.

The variant is one in which an arbitrary change has occurred in the amino acid sequence of the 'peptide', and may include one or more substitutions, deletions, additions and/or insertions. Such variants may be naturally occurring, or using any of a number of techniques well known in the art, it can be produced synthetically, for example by revising or modifying one or more of the peptide sequences of the present invention and evaluating their biological activity as described herein.

In one embodiment of the present invention, the variant includes conservative substitutions. A 'conservative substitution' is a substitution in which an amino acid is substituted with another amino acid having similar characteristics, and a person skilled in the art can predict that the secondary structure and sensitivity (hydropathic nature, hydrophobic or hydrophilic nature) of the peptide are substantially unchanged. In general, the following groups of amino acids exhibit conservative changes: (1) ala, pro, gly, glu, asp, gln, asn, ser, thr; (2) cys, ser, tyr, thr; (3) val, ile, leu, met, ala, phe; (4) lys, arg, his; and (5) phe, tyr, trp, his.

Modifications may be performed within the structure of the peptides of the present invention, yielding functional molecules that encode peptide variants or derivatives having desired (desirable) characteristics. In the case of changing the amino acid sequence of the peptide in order to prepare an equivalent or improved variant of the peptide of the present invention, one or more codons can be changed by those skilled in the art based on protein codon information known in the art.

Certain amino acids may be substituted for other amino acids within a protein or peptide structure without significant loss of the interactive binding capacity of the structure, eg, as a receptor, an antigen-binding site of an antibody, or a binding site on a substrate molecule. Because this is a commonly defined biological functional activity of a protein because of its interactive ability and properties, certain amino acid sequence substitutions can be made in (or within) protein or peptide sequences, it can also of course be made in (in) the underlying DNA coding sequence, and nevertheless obtain a protein having the same or similar properties.

Accordingly, it is contemplated that various changes be made to the disclosed peptide sequences or to the DNA sequences encoding the peptides without significant loss of desired utility or activity. In such modifications, the hydropathic (hydrophobic or hydrophilic nature) index of amino acids may also be considered. The importance of the hydropathic amino acid index in conferring interactive biological functions on proteins is generally understood in the art (Kyte and Doolittle, 1982, incorporated herein by reference). For example, the relative hydropathic nature of amino acids contributes to the secondary structure of the resulting protein, it is known that this in turn defines the interaction of the protein with other molecules such as enzymes, substrates, receptors, DNA, antibodies, antigens and the like. Each amino acid is assigned a hydropathic index based on its hydrophobicity and charge characteristics (Kyte and Doolittle, 1982). These values are: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamic acid (-3.5); glutamine (-3.5); aspartic acid (-3.5); asparagine (-3.5); lysine (-3.9); and arginine (-4.5).

It is known in the art that certain amino acids can be substituted by other amino acids having similar sensitivity indices or scores, and yielding proteins with similar biological activities (i.e. still obtain a biologically functionally equivalent protein). In this change, amino acids with a sensitivity index within ±2 are preferred for substitution, amino acid substitutions with a sensitivity index within ±1 are particularly preferred, and amino acid substitutions with a sensitivity index within ±0.5 are more particularly preferred.

It is also understood in the art that substitution of the same amino acid can be effectuated based on hydrophilicity. As described in U.S. Patent No. 4,554,101, the following hydrophilicity values are assigned to amino acid residues: arginine (+3.0); Lysine (+3.0); aspartic acid (+3.0±1); glutamic acid (+3.0±1); serine (+0.3); asparagine (+0.2); glutamine (+0.2); glycine (0); threonine (-0.4); proline (-0.5±1); alanine (-0.5); histidine (-0.5); cysteine (-1.0); methionine (-1.3); valine (-1.5); Leucine (-1.8); isoleucine (-1.8); tyrosine (-2.3); phenylalanine (-2.5); tryptophan (-3.4). It is understood that amino acids may be substituted with other amino acids having similar hydrophilicity values, and bioequivalent proteins may be obtained. In this change, substitution of an amino acid whose hydrophilicity value is within ±2 is preferred, substitution of an amino acid whose hydrophilicity value is within ±1 is particularly preferred, and substitution of an amino acid whose hydrophilicity value is within ±0.5 is even more particularly preferred.

As outlined above, amino acid substitutions can be based on the relative similarity of amino acid side-chain substituents, for example, it may be based on their hydrophobicity (relative similarity of side-chain substituents), hydrophilicity, charge, size, etc. Exemplary substitutions taking into account various features of the foregoing are well known to those skilled in the art and include: arginine and lysine; glutamic acid and aspartic acid; serine and threonine; glutamine and asparagine; valine, leucine and isoleucine.

Amino acid substitutions may also be made based on similarity in polarity, charge, solubility, hydrophobicity, hydrophilicity and/or amphipathic properties of the residues. For example, negatively charged amino acids include aspartic acid and glutamic acid; positively charged amino acids include lysine and arginine; and amino acids having uncharged polar head groups having similar hydrophilicity values include leucine, isoleucine and valine; glycine and alanine; asparagine and glutamine; serine, threonine, phenylalanine and tyrosine.

In addition, the variant may contain non-conservative modifications. In a preferred embodiment, variant peptides may differ from the native sequence by substitutions, deletions or additions of 5 or fewer amino acids. Variants can also be modified, for example by deletion or addition of amino acids that have minimal impact on the secondary structure and hydropathic properties of the peptide.

The peptide may include a signal (or leader) sequence at the N-terminus of the protein, this sequence directs transport of the protein either concurrently or post-translationally. The peptide may also be linked (conjugated) to a linker sequence or other sequence to facilitate synthesis, purification or identification (e.g. PolyHis) of the peptide, or to enhance binding of the peptide to a solid support. For example, the peptide may be linked (conjugated) to an immunoglobulin Fc region.

When peptide sequences are compared, when the two sequences are aligned in maximum correspondence as described later, if the amino acid sequences in the two sequences are the same, the two sequences are said to be "identical". Comparison between two sequences is typically performed by comparing sequences over a comparison window to identify and compare local regions of sequence similarity. The 'comparison window' used in this specification means a segment of at least about 20 consecutive positions, typically 30 to 75, 40 to 50 consecutive positions, in this segment, the sequence can be compared to a reference sequence of the same number of contiguous positions after the two sequences are optimally aligned.

Optimal alignment of sequences for comparison may be performed using default parameters, for example, using the Megalign program in the Lasergene suite of bioinformatics software (DNASTAR, Inc., Madison, Wis.). This program includes several alignment schemes described in the references below: Dayhoff, M. O. (1978) A model of evolutionary change in proteins-Matrices for detecting distant relationships. In Dayhoff, M. O. (ed.) Atlas of Protein Sequence and Structure, National Biomedical Research Foundation, Washington D.C. Vol. 5, Suppl. 3, pp. 345-358; Hein J. (1990) Unified Approach to Alignment and Phylogenes pp. 626-645 Methods in Enzymology vol. 183, Academic Press, Inc., San Diego, Calif.; Higgins, D. G. and Sharp, P. M. (1989) CABIOS 5:151-153; Myers, E. W. and Muller W. (1988) CABIOS 4:11-17; Robinson, E. D. (1971) Comb. Theor 11:105; Santou, N. Nes, M. (1987) Mol. Biol. Evol. 4:406-425; Sneath, P. H. A. and Sokal, R. R. (1973) Numerical Taxonomy-the Principles and Practice of Numerical Taxonomy, Freeman Press, San Francisco, Calif.; Wilbur, W. J. and Lipman, D. J. (1983) Proc. Nat'l Acad., Sci. USA 80:726-730.

Alternatively, optimal alignment of sequences for comparison is performed by the partial identity algorithm of Smith and Waterman (1981) Add.APL.Math 2:482, or performed by the identity alignment algorithm of Needleman and Wunsch (1970) J.Mol.Biol.48:443, or performed by the similarity search method of Pearson and Lipman (1988) Proc. Natl.Acad. Sci. USA 85:2444, or performed by computerized execution of these algorithms (GAP, BESTFIT, BLAST, FASTA, and TFASTA in the Wisconsin Genetics Software Package, Genetics Computer Group (GCG), 575 Science Dr., Madison, Wis.), or can be performed by inspection.

Examples of suitable algorithms for determining sequence identity and sequence similarity percentages may be the BLAST and BLAST2.0 algorithms, and these are described in Altschul et al. (1977) Nucl. Acids Res.25:3389-3402 and Altschul et al. (1990) J.Mol.Biol.215:403-410, respectively. BLAST and BLAST2.0 can be used to determine the percentage of sequence homology for the polynucleotides and polypeptides of the present invention, for example, with the parameters described herein. Software for performing BLAST analysis is publicly available through the National Center for Biotechnology Infomation. For amino acid sequences, a scoring matrix may be utilized to calculate a cumulative score.

The elongation of word hits in each direction stops in the following cases: when the cumulative alignment score has been reduced by a quantity X from its maximum, when the cumulative score has become zero or less due to the accumulation of one or more negatively scoring residues in the alignment, or when the end of any sequence has been reached. The BLAST algorithm parameters W, T, and X determine the sensitivity and speed of the alignment.

In one exemplary approach, the "percentage of sequence homology" is determined by comparing the two optimally aligned sequences over a comparison window of at least 20 positions, wherein a region of the polypeptide sequence in the comparison window may comprise no more than 20 percent, typically 5 to 15 percent, or 10 to 12 percent of additions or deletions (i.e., gaps) relative to the reference sequence (This does not include additions or deletions). The percentage is determined by determining the number of positions where the same amino acid residue is present in both sequences to obtain a number of matched positions, and the number of matched positions is divided by the total number of positions in the reference sequence (i.e., the window size), and the result is multiplied by 100 to yield a sequence homology percentage.

The peptides provided by the present invention can be in linear form or cyclic form, which can be understood with reference to the examples of the present invention.

The preparation of cyclic peptides as variants of the present invention is not particularly limited to, by any method of peptide cyclization known in the art, specific cyclization methods and the resulting cyclized forms. Preferably, the cyclized peptides of the present invention are prepared by taking a linear peptide and performing a cleavage or substitution such that a cysteine is located at both ends (N-terminal and C-terminal) thereof, and the preparation may be accomplished by causing a monosulfide bond to form between the cysteine residues present at both ends.

The peptides provided by the present invention, in one aspect, the use of modified polypeptides, wherein the modified polypeptides include modifications that improve the desired properties of the isolated polypeptides as described herein. Exemplary modifications of the polypeptides of the invention may include, but are not limited to, chemical derivatization and/or enzymatic derivatization at one or more constituent amino acids, wherein the derivatization includes side chain changes, backbone changes, and N-terminal and C-terminal changes, including acetylation, hydroxylation, methylation, amidation, and the addition of carbohydrate or lipid components, moieties, and the like. Exemplary modifications include pegylation of the polypeptide.

In certain aspects, chemoselective ligation techniques may be utilized to modify the peptides of the present invention, for example by attaching polymers in a site-specific and controlled manner. These techniques typically rely on the binding of a chemoselective anchor into the protein backbone, either by chemical means or recombinant means, and the subsequent modification with a polymer carrying a complementary linker. As a result, the assembly process and the covalent structure of the obtained protein-polymer conjugate are controlled, thereby enabling rational optimization of drug properties such as efficacy and pharmacokinetic properties. For example, by allowing the selective attachment of PEG, their pharmacokinetic properties are improved.

The peptides of the present invention may comprise a pharmaceutically acceptable salt form. Such pharmaceutically acceptable salts comprise, for example, but are not limited to, hydrochloride, sulfate, phosphate, acetate, citrate, tartrate, succinate, lactate, maleate, fumarate, oxalate, methanesulfonate, or paratoluenesulfonate.

The present invention also provides polynucleotides encoding the peptides.

As used herein, the terms 'DNA', 'polynucleotide', and 'nucleic acid' refer to DNA molecules that are isolated from the total genomic DNA of a particular species. Thus, a DNA fragment (segment) encoding a polypeptide refer to a DNA fragment comprising one or more coding sequences that are substantially isolated from, or purified from, the total genomic DNA of a species from which the DNA fragment can be obtained. The terms 'DNA fragment' and 'polynucleotide' include DNA fragments and smaller fragments of the fragments, and also include recombinant vectors (e.g., plasmids, cosmids, phagmids, bactericidal viruses, viruses, etc.).

As understood by those skilled in the art, the polynucleotide sequences of the present invention express, or are modified to express, proteins, peptides, and the like, and include genomic sequences, extra-genomic sequences, sequences encoded on plasmids, and smaller engineered gene fragments. These fragments may be isolated naturally or may be synthetically modified by humans.

As recognized by those skilled in the art, the polynucleotide may be single-stranded (coding sequence or antisense sequence), or may be double-stranded, and may be a DNA molecule (genomic, cDNA, or synthetic) or an RNA molecule. Additional coding or non-coding sequences may be present within the polynucleotides of the present invention. In addition, the polynucleotides may be linked to other molecules and/or support materials.

The polynucleotide may comprise a natural sequence, or may comprise a variant, or a biologically functional equivalent of the sequence. The polypeptide variant may comprise one or more substitutions, additions, deletions and/or insertions, such as those further described below, preferably such modifications are made such that the desired activity of the encoded polypeptide is not substantially reduced relative to the unmodified polypeptide. The effect on the activity of the encoded polypeptide can generally be evaluated as described herein.

The polynucleotides provided in the present invention are not particularly limited in their specific sequence and may have any combination of sequence (nucleic acid sequence) configurations, as long as they encode a peptide or variant peptide of the present invention. For example, a peptide comprising the amino acid sequence of SEQ ID NO: 2 may be expressed by a polynucleotide comprising the sequence represented by SEQ ID NO: 4, but is not limited thereto.

Regardless of the length of the coding sequence itself, the polynucleotides of the present invention may be combined with other DNA sequences, such as, for example, promoters, polyadenylation signals, additional restriction enzyme sites, multiple cloning sites, other coding fragments (parts, segments), and the like, with the result that their overall length may vary considerably. Thus, it is contemplated that polynucleotide fragments of virtually any length may be applicable, with their overall length preferably limited by ease of manufacture and use in the intended recombinant DNA protocol.

Furthermore, as a result of degeneracy of the genetic code, it will be self-evident to one of ordinary skill in the art that there are many nucleotide sequences encoding the peptides described herein. Several of these polynucleotides have minimal homology to the nucleotide sequence of any native gene. Nevertheless, due to differences in codon usage, other polynucleotides (e.g., polynucleotides optimized for human and/or primate codon selection) are specifically contemplated by the present invention.

In addition, alleles of a gene comprising a polynucleotide sequence provided herein are within the scope of the present invention. An allele is an endogenous gene that is modified as a result of one or more mutations, for example, deletions, additions and/or substitutions of nucleotides. The resulting mRNA and protein may (but may not necessarily) have an altered structure or function. Alleles may be identified using standard techniques (e.g., hybridization, amplification, and/or database sequence comparison).

Polynucleotides and fusions thereof may be prepared, engineered, and/or expressed using any of the well-established techniques known and available in the art. For example, a polynucleotide sequence encoding a peptide of the present invention, or a functional equivalent thereof, may be utilized within a recombinant DNA molecule directing expression of the polypeptide in an appropriate host cell. Due to the inherent degeneracy of the genetic code, other DNA sequences encoding substantially identical or functionally equivalent amino acid sequences may be generated, and these sequences can be cloned and utilized to express a given polypeptide.

As understood by those skilled in the art, in some instances it is advantageous to produce nucleotide sequences (nucleotide sequences encoding polypeptides) that possess codons that are not present in nature. For example, preferred codons in a particular prokaryotic or eukaryotic host may be selected to increase protein expression rates or produce recombinant RNA transcripts with desired properties (e.g., a half-life that is longer than the half-life of a transcript generated from a naturally occurring sequence).

In addition, the polynucleotide sequences of the present invention can be manipulated using methods generally known in the art to modify the peptide encoding sequence for a variety of reasons (including, but not limited to, alterations that modify the cloning, processing, expression and/or activity of the gene product).

The invention also provides a vector comprising the polynucleotide and a host cell transformed with the vector.

To express a polypeptide of interest, a nucleotide sequence encoding the polypeptide or functional equivalent can be inserted into a suitable expression vector (i.e., a vector containing the necessary elements for transcription and translation of the inserted coding sequence). Expression vectors comprising a sequence encoding the polypeptide of interest, and appropriate transcriptional control elements and translation control elements can be constructed by methods well known in the art. These methods include in vitro recombinant DNA techniques, synthetic techniques, and in vivo gene recombination: These methods are described in the following references Sambrook et al., Molecular Cloning, A Laboratory Manual(1989) and Ausubel et al., Current Protocols in Molecular Biology(1989).

Various expression vector/host systems are known in the art and can be utilized to contain and express polynucleotide sequences. Such expression vector/host systems include, but are not limited to, bacteria transformed with recombinant bacteriophage, plasmid, or cosmid DNA expression vectors; yeast transformed with yeast expression vectors; insect systems infected with viral expression vectors (e.g., baculoviruses); plant systems transformed with viral expression vectors (e.g., cauliflower mosaic virus, CaMV; tobacco mosaic virus, TMV) or bacterial expression vectors (e.g., Ti or pBR322 plasmids); or microorganisms, such as animal systems.

The 'control elements' or 'regulatory sequences' present within an expression vector are non-translational regions (enhancers, promoters, 5' and 3' non-translational regions) that interact with host cell proteins to execute transcription and translation. These elements can vary in strength and specificity. Depending on the vector system and host used, any number of appropriate transcriptional and translational elements (including constitutive promoters and inducible promoters) can be utilized.

For example, when cloning into bacterial systems, inducible promoters such as the PBLUESCRIPT phage (Stratagene, La Jolla, Calif.) or the hybridized lacZ promoter from the PSPORT1 plasmid (Gibco BRL, Gaithersburg, MD) can be used. In mammalian systems, promoters derived from mammalian genes or mammalian viruses are generally preferred. If it is necessary to generate cell lines containing multiple copies of the sequence encoding the polypeptide, SV40 or EBV-based vectors may be useful in combination with appropriate selection markers.

In bacterial systems, a number of expression vectors can be selected for peptide expression depending on the intended use. For example, if large quantities are required, vectors directed toward high levels of expression of easily purified fusion proteins may be utilized. Such vectors include, but are not limited to, the following: Multifunctional E.coli cloning vectors and expression vectors, such as BLUESCRIPT (Stratagene), in which the sequence encoding the polypeptide of interest is linked in the vector in-frame with the sequence for the amino-terminal Met and seven subsequent residues of β-galactosidase, resulting in the production of a hybrid protein; pIN vectors (Van Heeke and Schuster, J.Biol.Chem.264:5503 5509 (1989)); etc. The pGEX vector (Promega, Madison, Wis.) can also be utilized to express foreign polypeptides as fusion proteins with glutathione S-transferase (GST). Typically, such fusion proteins are soluble and can be readily purified from lysed cells by adsorption onto glutathione-agarose beads and subsequent elution in the presence of free glutathione. Proteins produced in this system can be designed to include heparin, thrombin, or Factor Xa protease cleavage sites to allow the cloned polypeptide to be released from the GST moiety.

In yeast (*Saccharomyces cerevisiae*), a number of vectors can be utilized, including constitutive promoters or inducible promoters (e.g., alpha-factor, alcohol oxidase, and PGH). This can be understood by reference to Ausubel et al (supra). and Grant et al., Methods Enzymol.153:516-544 (1987), among others.

When utilizing a plant expression vector, expression of the sequence encoding the polypeptide can be driven by any number of promoters. For example, a viral promoter (e.g., the 35S promoter and 19S promoter of CaMV) can be used alone or in combination with an ω (omega) leader sequence derived from TMV (Takamatsu, EMBO J.6:307-311 (1987)). Alternatively, a plant promoter (e.g., a small subunit of RUBISCO or a heat shock promoter) can be utilized. These constructs can be introduced into plant cells by direct DNA transformation or pathogen-mediated transfection. Such techniques are known in the art.

Insect systems can also be utilized to express targeted polypeptides. For example, in one system, Autographa californica nuclear polyhedrosis virus (AcNPV) is utilized as a vector to express a foreign gene in *Spodoptera frugiperda* cells or *Trichoplusia larvae.* The sequence encoding the polypeptide can be cloned within a non-essential region of the virus and located under the control of a polyhedrin promoter, for example, a polyhedrin gene. Successful insertion of the sequence encoding the polypeptide renders the polyhedrin gene inactive and produces a recombinant virus lacking the coat protein. The recombinant virus can then be used to infect, for example, *S*. *frugiperda* cells or *Trichoplusia larvae,* where the polypeptide of interest is expressed.

In mammalian host cells, a number of virus-based expression systems are commonly available. For example, when an adenovirus is utilized as an expression vector, the sequence encoding the polypeptide of interest can be linked within an adenoviral transcription/translation complex consisting of a late promoter and a tripartite leader sequence. By utilizing insertions into non-essential E1 or E3 regions of the viral genome, a viable virus capable of expressing the polypeptide in an infected host cell can be obtained. Transcriptional enhancers (e.g., Rous Sarcoma Virus (RSV) enhancers) may also be utilized to increase expression in mammalian host cells.

In addition, certain initiation signals may be utilized for more efficient translation of the sequence encoding the polypeptide of interest. Examples of such signals include the ATG start codon and neighboring sequences. If the sequence encoding the polypeptide, its start codon, and upstream sequences are inserted into a suitable expression vector, no additional transcriptional or translation control signals may be required. However, if only the coding sequence, or a portion thereof, is inserted, an exogenous translation control signal containing the ATG start codon must be provided. The start codon must also be within the correct reading frame, to ensure translation of the entire insertion. Exogenous translation elements and start codons can be of various origins (both natural and synthetic). The efficiency of expression can be augmented by the inclusion of appropriate enhancers for the specific cell system utilized.

The host cell line may also be selected based on its ability to regulate the expression of the inserted sequence and/or to process the expressed protein in a desired manner. Such modifications of polypeptides include, but are not limited to, acetylation, carboxylation, glycosylation, phosphorylation, lipidation, and acylation. Post-translational processing that cleaves the 'prepro' form of a protein may be utilized to facilitate correct insertion, folding, and/or function. Different host cells (e.g. CHO, HeLa, MDCK, HEK293 and W138, which have specific cellular machinery and characteristic mechanisms for such post-translational activities) may be selected to ensure accurate modification and processing of the foreign protein.

In the long term, for high yield production of a recombinant protein, stable expression is generally desirable. For example, a cell line stably expressing a polynucleotide of interest may be transformed using an expression vector, wherein the expression vector may comprise a viral replication site and/or an endogenous expression element, and a selection marker gene on the same vector or on a separate vector.

After introduction of the vector, the cells may be grown in enriched media for 1 to 2 days, after which they are converted to selective media. The purpose of the selection marker is to confer resistance to selection, and its presence allows for the growth and recovery of cells that are successfully expressing the introduced sequence. Resistant clones of stably transformed cells can be grown using tissue culture techniques appropriate for the cell type.

A number of selection systems can be used to recover transformed cell lines. These selection systems include, but are not limited to, the herpes simplex virus thymidine kinase and adenine phosphoribosyl transferase genes, which can be used for tk-cells or aprt-cells, respectively.

In addition, antimetabolite resistance, antibiotic resistance, or herbicide resistance can be used as a basis for selection. Examples include dhfr conferring resistance to methotrexate; npt conferring resistance to aminoglycosides, neomycin, and G-418; and als or pat conferring resistance to chlorsulfuron and phosphinotricin acetyltransferase, respectively (see Murry, supra). Additional selection genes are known in the art, such as trpB, which enables cells to utilize indole instead of tryptophan, or hisD, which enables cells to utilize histinol instead of histidine. Visible markers such as anthocyanins, β-glucuronidase and its substrate GUS, and luciferase and its substrate luciferin are gaining popularity, and widely used not only to identify transformants but also to quantify the amount of transient or stable protein expression attributable to a particular vector system.

Various protocols are known in the art for detecting and measuring the expression of a product encoded by a polynucleotide, using either polyclonal or monoclonal antibodies specific for the product encoded by the polynucleotide. Examples include enzyme-linked immunosorbent assay (ELISA), radioimmunoassay (RIA), and fluorescence activated cell sorting (FACS). These and additional assay methods can be found in methods known in the art, which are hereby incorporated by reference. Various labeling techniques and conjugation techniques are known in the art and can be utilized for various nucleic acid assays and amino acid assays. Means for producing labeled hybridization probes or labeled PCR probes to detect sequences associated with polynucleotides include oligolabeling, nick translation, terminal labeling, or PCR amplification using labeled nucleotides. Alternatively, for the production of an mRNA probe, the sequence or any portion thereof may be cloned into a vector. Such vectors are known and commercially available in the art and can be used to synthesize RNA probes in vivo by adding an appropriate RNA polymerase (e.g., T7, T3, or SP6) and labeled nucleotides. These procedures can be performed using a variety of commercially available kits. Suitable reporter molecules or labels include radionuclides, enzymes, fluorophores, chemiluminescent or chromogenic agents, substrates, promoters, inhibitors, and magnetic particles.

Host cells transformed with the polynucleotide sequence of interest may be cultured under conditions suitable for the expression of the protein and its recovery from the cell culture. The protein produced by the recombinant cell may be secreted or contained within the cell, depending on its sequence and/or vector.

As understood by those skilled in the art, an expression vector comprising a polynucleotide of the present invention can be designed to include a signal sequence directing the secretion of a polypeptide through a prokaryotic or eukaryotic cell membrane. Other recombinant constructs may be utilized to link the sequence encoding the polypeptide of interest to the sequence encoding the polypeptide domain that facilitates purification of the soluble protein.

In addition to recombinant production methods, the polypeptides and fragments thereof of the present invention can be produced by direct peptide synthesis using solid phase techniques. Protein synthesis can be performed using manual techniques or by automation. Automated synthesis can be achieved, for example, using an Applied Biosystems 431 A Peptide Syntthesizer (Perkin Elmer). Alternatively, the various fragments can be chemically synthesized individually and then combined using chemical methods to produce a full length molecule.

According to another aspect of the present invention, a polynucleotide encoding a polypeptide of the present invention can be delivered to a subject in vivo, for example, using gene therapy techniques. Gene therapy generally refers to the introduction of a heterologous nucleic acid into a target cell, a specific cell of a mammal, particularly a human, that has a disorder or symptom requiring such treatment. The nucleic acid is introduced into the selected target cell, the heterologous DNA is expressed, and the therapeutic product encoded by it is produced.

Various viral vectors that can be used for gene therapy as disclosed herein include RNA viruses such as adenoviruses, herpes viruses, vaccinia, adeno-associated virus (AAV), or preferably retroviruses. Preferably, the retroviral vector may be a murine or avian retroviral derivative, or a lentiviral vector. Preferred retroviral vectors may be lentiviral vectors. Examples of retroviral vectors into which a single exogenous gene may be inserted include, but are not limited to, the following: Moloney murine leukemia virus (MoMuLV), Harvey murine sarcoma virus (HaMuSV), murine mammary tumor virus (MuMTV), SIV, BIV, HIV and Rous Sarcoma Virus (RSV). Many additional retroviral vectors can incorporate multiple genes. All of these vectors can include a selection marker gene to ensure that transduced cells are identified and produced. For example, a targeted zinc finger-derived DNA binding polypeptide sequence can be inserted into a viral vector along with another gene encoding a ligand for a receptor on a specific target cell, thereby making the vector target specific.

The retroviral vector can be made target specific, for example, by inserting polynucleotides encoding a polypeptide. For example, targeting can be achieved by using an antibody directed against the retroviral vector. Those skilled in the art are familiar with specific polynucleotide sequences that can be inserted into a retroviral genome to enable target-specific delivery of a retroviral vector comprising a zinc finger-nucleotide binding protein polynucleotide, which can be readily identified without undue experimentation.

Since recombinant retroviruses are defective, they require assistance to produce infectious vector particles. This assistance can be provided, for example, by using a helper cell line containing plasmids encoding all the structural genes of the retrovirus under the control of regulatory sequences in the LTR. These plasmids lack the nucleotide sequences that enable the packaging mechanism to recognize RNA transcripts for encapsulation. Helper cell lines lacking packaging signaling include, but are not limited to, PSI.2, PA317, and PA12. These cell lines produce empty virions because the genome is not packaged. If a retroviral vector is introduced into cells in which the packaging signal is intact and the structural gene has been replaced by another gene of interest, the vector can be packaged and vector virions produced. The vector virions produced by the above methods can then be used to infect tissue cell lines (e.g., NIH3T3 cells), thereby producing large quantities of chimeric retroviral virions.

In addition, 'non-viral' delivery techniques for gene therapy may be utilized, such as DNA-ligand complexes, adenovirus-ligand-DNA complexes, direct injection of DNA, CaPO4 precipitation, gene gun technology, electroporation, liposome technology, and lipofection. Any of these methods are widely available to those skilled in the art and are suitable for use in the present invention. Other suitable methods are also available to those skilled in the art, and it will be self-evident that the present invention can be accomplished using any available transfection method. Lipofection can be achieved by encapsulating isolated DNA molecules within liposomal particles and contacting said liposomal particles with the cell membrane of a target cell. Liposomes are colloidal, self-assembling molecules in which a lipid bilayer composed of amphiphilic molecules such as phosphatidylserine or phosphatidylcholine encapsulates a portion of the surrounding media, resulting in a lipid bilayer surrounding a hydrophilic interior. Unilamellar or multilamellar liposomes can be constructed, resulting in an interior containing the desired chemical, drug, or isolated DNA molecule, such as in the present invention.

The present invention also provides A vaccine adjuvant comprising at least one selected from the group consisting of the following (i) to (iv).
(i) the peptide,
(ii) a polynucleotide encoding (i) the peptide;
(iii) a vector comprising (ii) the polynucleotide, and
(iv) a host cell transformed by (iii) the vector.:
   In the present invention, the vaccine adjuvant may be defined as a substance capable of presenting an antigen to the immune system in such a way that an immune response to the antigen, or an increase in the immune response, is induced when the antigen is administered with said vaccine adjuvant. In other words, the vaccine adjuvant is an immune enhancer, meaning a substance that promotes an immune response to an antigen, and is not an immunogen to the host, but enhances immunity by increasing the activity of cells of the immune system.

To analyze an antigen-specific induced immune response by the vaccine adjuvant, the immune response may be compared to an immune response induced in the presence of the antigen without the vaccine adjuvant. The induction may be assessed in a subject or in cells from a subject.

In the present invention, the immune response may be at least one selected from the group consisting of macrophage, dendritic cell, monocyte, B cell, and T cell responses. In particular, the immune response may be a B cell response, meaning that it is capable of producing antibodies specifically against the antigen. The antibody is preferably an IgG antibody, more preferably an IgG2a and/or an IgG1 antibody. The immune response may be a T cell response, preferably a Th1 response, a Th2 response or a balanced Th2/Th1 response. One of ordinary skill in the art will recognize that disease-dependent B and/or T cell responses may need to be induced to regulate them. Variably, the immune response may be detected by measuring, for example, the production of cytokines such as IFNgamma, IL-6, TNFalpha, or IL-10. The production of these cytokines may be assessed by ELISA, preferably as performed in an embodiment.

According to one embodiment of the present invention, the peptide of SEQ ID NO: 2 has been found to have a very good effect of activating both innate and acquired immunity, so that the increase in the immune response of the subject can be significantly enhanced when treated with the antigen.

Thus, it will be self-evident to a person of ordinary skill in the art that a peptide of SEQ ID NO: 2; or a peptide comprising an amino acid sequence exhibiting at least 95% homology to the peptide, a polynucleotide encoding said polynucleotide, a vector comprising the polynucleotide, and a host cell transformed with the vector will also exhibit the same physiological activity.

In one embodiment of the present invention, the vaccine adjuvant may induce the activation of improved innate and acquired immune responses, and more specifically, improved cellular and humoral immune responses, when administered with the antigen and the vaccine adjuvant compared to the antigen alone.

In one embodiment of the invention, detection of the antigen-specific induced immune response means that the detection occurs at least 1, 2, 3, 4, 5, 6, 7, 8, 9, 10, 11, 12, or more hours after administration of the vaccine adjuvant of the invention, or at least 1 day after, or at least 2 days after, or at least 3 days after, or at least 4 days after, or more hours after. The detection may be assessed in a subject or in cells of a subject, preferably as performed in an embodiment.

In one embodiment of the invention, the antigen-specific induced immune response preferably means a detectable immune response to the antigen. The detectable increase may occur at least 1, 2, 3, 4, 5, 6, 7, 8, 9, 10, 11, 12 hours after administration of the vaccine adjuvant and antigen to the subject, or after a longer period of time, or at least 1 day after, or at least 2 days after, or at least 3 days after, or at least 4 days after, or a longer period of time after, may mean an increase of at least 5%, or 10%, 15%, 20%, 25%, 30%, 35%, 40%, 45%, 50%, 55%, 60%, 65%, 70%, 75%, 80%, 85%, 90%, 95%, 100%, 150%, 200%, or more in the amount of immune cells, antibodies, and/or cytokines. The detection may be assessed in a subject or in cells of a subject, preferably as performed in an embodiment.

The present invention also provides a vaccine composition comprising the vaccine adjuvant and antigen.

In the present invention, the vaccine is a preparation containing an antigen comprising a whole disease-causing organism (hereinafter rescued or attenuated) or a component of such an organism, such as a protein, peptide or polysaccharide, and used to confer immunity to a disease caused by the organism. As described above, a peptide comprising the amino acid sequence of SEQ ID NO: 2; or a peptide comprising an amino acid sequence exhibiting at least 95% homology to the peptide may be utilized as a vaccine composition by activating the innate and acquired immune systems to significantly augment the immune response of a subject when administered with an antigen.

In the present invention, the antigens included in the vaccine composition may be, but are not limited to, peptides, proteins, glycoproteins, glycolipids, lipids, carbohydrates, nucleic acids, polysaccharides, and viruses, fungi, allergens, tissues, cells, and the like. Non-limiting examples include pollen-derived antigens, hepatitis A virus-derived antigens, hepatitis B virus-derived antigens, Hepatitis A virus-derived antigens, Hepatitis B virus-derived antigens, Hepatitis C virus-derived antigens, Hepatitis D virus-derived antigens, Hepatitis E virus-derived antigens, Hepatitis F virus-derived antigens, HIV virus-derived antigens, Influenza virus-derived antigens, Herpes virus (HSV-1, HSV-2) derived antigens, anthrax-derived antigen, chlamydia-derived antigen, pneumococcal-derived antigen, Japanese encephalitis virus-derived antigen, measles virus-derived antigen, rubella virus-derived antigen, tetanus bacteria-derived antigen, varicella virus-derived antigen, SARS virus-derived antigen, EB virus-derived antigen, Papilloma virus-derived antigen, *Helicobacter pylori* bacteria-derived antigen, Rabies virus-derived antigen, West Nile virus-derived antigen, Hanta virus-derived antigen, Streptococcus pneumoniae-derived antigen, *Staphylococcus* aureus-derived antigen, *Bordetella pertussis-derived* antigens, *Mycobacterium tuberculosis-derived* antigens, Plasmodium-derived antigens, Poliovirus-derived antigens, various zoonotic infections, cancer antigens, and food allergy-derived antigens and the like.

The antigens included in the vaccine compositions of the present invention need not be single. This is because in some applications of the present invention, an immune response may be generated against a plurality of components, such as cancer cells, bacteria, or viruses, rather than a single protein or peptide, in this case, it may be a plurality of proteins, etc. that can cause an immune response, or a mixture that cannot be specified. It is also possible to use the vaccine composition of the present invention to actively produce an immune response to a plurality of antigens, and to comprise a plurality of antigens.

The antigen included in the vaccine composition of the present invention may preferably be a tumor antigen. A tumor antigen is a tumor specific antigen (TSA) or a tumor associated antigen (TAA). Several tumor antigens and their expression patterns are known in the art and can be selected depending on the type of tumor to be treated. Non-limiting examples of tumor antigens include alpha fetoprotein, carcinoembryonic antigen, cdk4, beta-catenin, CA125, caspase-8, epithelial tumor antigen, HPV antigen, HPV16 antigen, CTL epitope derived from HPV16 E7 antigen, melanoma associated antigen (MAGE)-1, MAGE-3, tyrosinase , surface Ig idiotype, Her-2/neu, MUC-1, prostate specific antigen (PSA), sialyl Tn (STn), heat shock protein, gp96, ganglioside molecules GM2, GD2, GD3, carcinoembryonic antigen (CEA), PRAME, WT1, survivin, cyclin D, cyclin E, HER2, MAGE, NY-ESO, EGF, GP100, cathepsin G, human papillomavirus (HPV)-16- E6, HPV-16-E7, HPV-18-E6, HPV-18-E7, Her/2-neu antigen, chimeric Her2 antigen, prostate specific antigen (PSA), bivalent PSA, ERG, androgen receptor (AR), PAK6, prostate stem cell antigen (PSCA), NY-ESO-1, Stratum Corneum Chymotryptic Enzyme (SCCE) antigen, Wilms tumor antigen 1 (WT-1), HIV-1 Gag, Human Telomerase Reverse Transcriptase (hTERT), proteinase 3, tyrosinase-related protein 2 (TRP2), high molecular weight melanoma-associated antigen (HMW-MAA), synovial sarcoma, X (SSX)-2, carcinoembryonic antigen (CEA), melanoma-associated antigen E (MAGE-A, MAGE1, MAGE2, MAGE3, MAGE4), interleukin-13 receptor alpha (IL13-R alpha), carbonic anhydrase IX (CAIX), survivin, GP100, angiogenic antigen, ras protein, p53 protein, p97 melanoma antigen, KLH antigen, carcinoembryonic antigen (CEA), gp100, MART1 antigen, TRP-2, HSP-70, beta-HCG, testicine, 1A01_HLA-A/m; 1A02; 5T4; ACRBP; AFP; AKAP4; alpha-actinin-_4/m; alpha-methylacyl-coenzyme_A_racemase; ANDR; ART-4; ARTC1/m; AURKB; B2MG; B3GN5; B4GN1; B7H4; BAGE-1; BASI; BCL-2; bcr/abl; beta-catenin/m; BING-4; BIRC7; BRCA1/m; BY55; calreticulin; CAMEL; CASPA; caspase_8; cathepsin_B; cathepsin_L; CD1A; CD1B; CD1C; CD1D; CD1E; CD20; CD22; CD276; CD33; CD3E; CD3Z; CD4; CD44_isoform_1; CD44_isoform_6; CD52; CD55; CD56; CD80; CD86; CD8A; CDC27/m; CDE30; CDK4/m; CDKN2A/m; CEA; CEAM6; CH3L2; CLCA2; CML28; CML66; COA-1/m; coactosin-like_protein; collagen_XXIII; COX-2; CP1 B1; CSAG2; CT-_9/BRD6; CT45A1; CT55; CTAG2_isoform_LAGE-1A; CTAG2_isoform_LAGE-1B; CTCFL; Cten; cyclin_B1; cyclin_D1; cyp-B; DAM-10; DEP1A; E7; EF1A2; EFTUD2/m; EGFR; EGLN3; ELF2/m; EMMPRIN; EpCam; EphA2; EphA3; ErbB3; ERBB4; ERG; ETV6; EWS; EZH2; FABP7; FCGR3A_version_1; FCGR3A_version_2; FGF5; FGFR2; fibronectin; FOS; FOXP3; FUT1; G250; GAGE-1; GAGE-2; GAGE-3; GAGE-4; GAGE-5; GAGE-6; GAGE7b; GAGE-8_ (GAGE-2D); GASR; GnT-V; GPC3; GPNMB/m; GRM3; HAGE; hepsin; Her2/neu; HLA-A2/m; Homeobox_NKX3.1; HOM-TES-85; HPG1; HS71A; HS71B; HST-2; hTERT; iCE; IF2B3; IL-10; IL-13Ra2; IL2-RA; IL2-RB; IL2-RG; IL-5; IMP3; ITA5; ITB1; ITB6; kallikrein-2; kallikrein-4; KI20A; KIAA0205; KIF2C; KK-LC-1; LDLR; LGMN; LIRB2; LY6K; MAGA5; MAGA8; MAGAB; MAGE-_B1; MAGE-_E1; MAGE-A1; MAGE-A10; MAGE-A12; MAGE-A2; MAGE-A3; MAGE-A4; MAGE-A6; MAGE-A9; MAGE-B10; MAGE-B16; MAGE-B17; MAGE-B2; MAGE-B3; MAGE-B4; MAGE-B5; MAGE-B6; MAGE-C1; MAGE-C2; MAGE-C3; MAGE-D1; MAGE-D2; MAGE-D4; MAGE-E1_(MAGE1); MAGE-E2; MAGE-F1; MAGE-H1; MAGEL2; mammaglobin_A; MART-1/melan-A; MART-2; MC1_R; M-CSF; mesothelin; MITF; MMP1_1; MMP7; MUC-1; MUM-1/m; MUM-2/m; MYO1A; MYO1B; MYO1C; MYO1D; MYO1E; MYO1F; MYO1G; MYO1H; NA17; NA88-A; Neo-PAP; NFYC/m; NGEP; N-myc; NPM; NRCAMs; NSE; NUF2; NY-ESO-1; OA1; OGT; OS-9; osteocalcin; osteopontin; p53; PAGE-4; PAI-1; PAI-2; PAP; PATE; PAX3; PAX5; PD1L1; PDCD1; PDEF; PECA1; PGCB; PGFRB; Pim-1_-kinase; Pin-1; PLAC1; PMEL; PML; POTE; POTEF; PRAME; PRDX5/m; PRM2; prostein; proteinase-3; PSA; PSB9; PSCA; PSGR; PSM; PTPRC; RAB8A; RAGE-1; RARA; RASH; RASK; RASN; RGS5; RHAMM/CD168; RHOC; RSSA; RU1; RU2; RUNX1; S-100; SAGE; SART-1; SART-2; SART-3; SEPR; SERPINB5; SIA7F; SIA8A; SIAT9; SIRT2/m; SOX10; SP17; SPNXA; SPXN3; SSX-1; SSX-2; SSX3; SSX-4; ST1A1; STAG2; STAMP-1; STEAP-1; survivin; survivin-2B; SYCP1; SYT-SSX-1; SYT-SSX-2; TARP; TCRg; TF2AA; TGFbeta1; TGFR2; TGM-4; TIE2; TKTL1; TPI/m; TRGV11; TRGV9; TRPC1; TRP-p8; TSG10; TSPY1; TVC_(TRGV3); TX101; tyrosinase; TYRP1; TYRP2; UPA; VEGFR1; WT1; XAGE1, alpha-actinin-4; ARTC1; BCR-ABL fusion protein (b3a2); B-RAF; CASP-5; CASP-8; beta-catenin; Cdc27; CDK4; CDKN2A; COA-1; dek-can fusion protein; EFTUD2; Elongation factor 2; ETV6-AML1 fusion protein; FN1; GPNMB; LDLR-fucosyltransferase AS fusion protein; HLA-A2d; HLA-A11d; hsp70-2; KIAAO205; MART2; ME1; MUM-If; MUM-2; MUM-3; neo-PAP; myosin class I; NFYC; OGT; OS-9; pml-RARalpha fusion protein; PRDX5; PTPRK; K-ras; N-ras; RBAF600; SIRT2; SNRPD1; SYT-SSX1 or SSX2 fusion protein; Triosephosphate Isomerase; BAGE-1; GAGE-1,2,8; GAGE-3,4,5,6,7; GnTVf; HERV-K-MEL; KK-LC-1; KM-HN-1; LAGE-1; MAGE-A1; MAGE-A2; MAGE-A3; MAGE-A4; MAGE-A6; MAGE-A9; MAGE-A10; MAGE-A12; MAGE-C2; mucin k; NA-88; NY-ESO-1/LAGE-2; SAGE; Sp17; SSX-2; SSX-4; TRAG-3; TRP2-INT2g; CEA; gp100/Pmel17; Kallikrein 4; mammaglobin-A; Melan-A / MART-1; NY-BR-1; OA1; PSA; RAB38/NY-MEL-1; TRP-1/gp75; TRP-2; tyrosinase; adipophilin; AIM-2; BING-4; CPSF; cyclin D1; Ep-CAM; EphA3; FGF5; G250/MN/CAIX; HER-2/neu; IL13Ralpha2; Intestinal carboxyl esterase; Alpha-foetoprotein; M-CSF; mdm-2; MMP-2; MUC1; p53; PBF; PRAME; PSMA; RAGE-1; RNF43; RU2AS; secernin 1; SOX10; STEAP1; survivin; telomerase; WT1; FLT3-ITD; BCLX(L); DKK1; ENAH (hMena); MCSP; RGS5; gastrin-17; Human Chorionic Gonadotropin, EGFRvIII, HER2, HER2/neu, P501, Guanylyl Cyclase C, prostatic acid phosphatase (PAP), ovalbumin (OVA) and MART-1.

The vaccine composition of the present invention may preferably be an anti-cancer vaccine. In addition, the anti-cancer vaccine may be a vaccine for preventing cancer or a vaccine for treating cancer.

According to an aspect of the invention, a vaccine composition comprising a peptide comprising the amino acid sequence of SEQ ID NO: 2 (or a peptide comprising an amino acid sequence exhibiting at least 95% sequence homology thereto) and a specific tumor antigen can be administered to a subject prior to the formation of a cancer and exhibit a prophylactic effect of inhibiting the growth of the cancer by activating the immune response of the subject. In addition, the vaccine compositions of the present invention have been shown to be effective as therapeutic vaccines when administered to a subject after the formation of cancer to inhibit the growth of cancer and/or kill cancer.

In the present invention, the type of cancer is not particularly limited and may be preferably selected from the group consisting of breast cancer, colorectal cancer, prostate cancer, cervical cancer, stomach cancer, skin cancer, head and neck cancer, lung cancer, glioblastoma, oral cancer, pituitary adenoma, glioma, brain tumor, pharyngeal cancer, laryngeal cancer, thymoma, mesothelioma, esophageal cancer, rectal cancer, liver cancer, pancreatic cancer, pancreas endocrine tumors, gallbladder cancer, penile cancer, ureter cancer, renal cell cancer, bladder cancer, non-Hodgkin's lymphoma, myelodysplastic syndrome, multiple myeloma, plasma cell tumor, leukemia, childhood cancer, bronchial cancer, colon and ovarian cancer.

The vaccine composition of the present invention may further include at least one selected from the group consisting of an arbitrary vaccine adjuvant and an immune checkpoint inhibitor.

In one embodiment of the present invention, it has been found that the peptides of the present invention have a more pronounced effect on activating the immune function of a subject when treated in combination with any additional vaccine adjuvant and/or immune checkpoint inhibitor. In particular, this effect may be particularly desirable in that it minimizes side effects that may be caused by administering high doses of the substance, and allows for maximum effectiveness to be achieved with minimal administration.

The type of vaccine adjuvant that may be further comprised in the vaccine composition of the present invention is not particularly limited, and it should be understood that vaccine adjuvants currently widely used in the art or vaccine adjuvants to be newly developed in the future are also included therein. As a non-limiting example of any of the above vaccine adjuvants may include 1018 ISS, Aluminum Salt, Amplivax, AS15, BCG, CP-870,893, CpG ODN, CpG7909, SIA, dSLIM, GM-CSF, IC30, IC31, Lmiquimod, Imufact IMP321, IS Patch, Iscomatrix, Juvlmmune, Lipovac, MF59, Monophosphoryl Lipid A, Montanide IMS 1312, Montanide ISA 206, Montanide ISA 50V, Montanide ISA-51, OK-432, OM-174, OM-197-MP-EC, ONTAK, PepTel Vector System, PLG Microparticles, Resiquimod, SRL172, Virosomes and other Virus-like Particles, YF-17DBCG, Aquila's QS21 stimulon, Ribi's Detox. Quil, Superfos, Freund's, GM-CSF, Cholera Toxins, Immunological Adjuvants, MF59 and Cytokines and most preferably, it may be CpG ODN.

In the present invention, said immune checkpoint inhibitor is a substance that blocks an immune system inhibitor checkpoint. Immune checkpoints can be either stimulatory or inhibitory. Blockade of inhibitory immune checkpoint activates immune system function and can be used in cancer immunotherapy [see, e.g., Pardoll, Nature Reviews. Cancer 12:252-64 (2012)]. Tumor cells, when they attach to specific T-cell receptors, cause a loss of activated T cells. Immune checkpoint inhibitors prevent tumor cells from attaching to T cells, which keeps the T cells active. In fact, the cooperative actions of cells and soluble components combat the damage caused by pathogens and cancer. Modulation of an immune system checkpoint entails altering the expression or functional activity of at least one component of the pathway, thereby modulating the response by the immune system. The immune checkpoint inhibitor may be selected from the group consisting of programmed cell death-1 (PD-1) antagonists, a programmed cell death-ligand 1 (PD-L1) antagonist, programmed cell death-ligand 2 (PD-L2) antagonist, cluster of differentiation 27 (CD27) antagonist, cluster of differentiation 28 (CD28) antagonist, cluster of differentiation 70 (CD70) antagonist, cluster of differentiation 80, also known as B7-1 (CD80) antagonist, cluster of differentiation 86, also known as B7-2 (CD86) antagonist, cluster of differentiation 137 (CD137) antagonist, cluster of differentiation 276 (CD276) antagonist, killer-cell immunoglobulin-like receptors (KIRs) antagonist, lymphocyte-activation gene 3 (LAG3) antagonist, tumor necrosis factor receptor superfamily, member 4, also known as CD134 (TNFRSF4) antagonist, glucocorticoid-induced TNFR-related protein (GITR) antagonist, glucocorticoid-induced TNFR- related protein ligand (GITRL) antagonist, 4-1BB ligand (4-1BBL) antagonist, cytolytic T lymphocyte associated antigen-4 (CTLA-4) antagonist, Adenosine A2A receptor (A2AR) antagonist, V-set domain-containing T-cell activation inhibitor 1 (VTCN1) antagonist, B- and T-lymphocyte attenuator (BTLA) antagonist, indoleamine 2,3-dioxygenase (IDO) antagonist, T-cell Immunoglobulin domain and Mucindomain (3TIM-3) antagonist, V-domain Ig suppressor of T cell activation (VISTA) antagonists and killer cell lectin-like receptor subfamilyA (KLRA) antagonists, and may preferably be a PD-1 antagonist, a PD-L1 antagonist or a LAG3 antagonist, most preferably a PD-L1 antagonist.

In one embodiment of the invention, the immune checkpoint inhibitor may be a PD-1 antagonist. The PD-1 is a T-cell costimulatory receptor that plays a central role in the ability of tumor cells to evade the host's immune system. Blockade of the interaction between PD-1 and PD-L1, the ligand of a PD-1 antagonist, enhances immune function and mediates antitumor activity. Examples of the PD-1 antagonists include antibodies that specifically bind to PD-1. Specific anti-PD-1 antibodies include, but are not limited to, nivolumab, pembrolizumab, STI-1014, and fidilumab.

In another embodiment of the present invention, the immune checkpoint inhibitor may be a PD-L1 antagonist. Examples of the PD-L1 antagonists include antibodies that specifically bind to PD-L1. Specific anti-PD-L1 antibodies include, but are not limited to, avelumab, atezolizumab, durvalumab, and BMS-936559.

In another embodiment, the immune checkpoint inhibitor may be a LAG3 antagonist. LAG3, lymphocyte activator gene 3, is a negative co-stimulatory receptor that regulates T cell homeostasis, proliferation, and activation. LAG3 has also been reported to be involved in regulatory T cell (Treg) suppressive functions. The majority of LAG3 molecules are retained in cells proximal to microtubule-organizing centers and are induced only after antigen-specific T cell activation [ref: U.S. 2014/0286935]. Examples of LAG3 antagonists include antibodies that specifically bind to LAG3. Specific anti-LAG3 antibodies include, but are not limited to, GSK2831781.

In the present invention, the antibodies are meant to comprise intact monoclonal antibodies, polyclonal antibodies, multi-specific antibodies and antibody fragments formed from at least two intact antibodies, insofar as they exhibit the biological activity for which they are intended. In another embodiment, the antibody comprises a soluble receptor that does not retain the Fc portion of the antibody. In one embodiment, the antibody may be a humanized monoclonal antibody and fragment thereof produced by recombinant genetic manipulation.

Another class of immune checkpoint inhibitors includes polypeptides that bind to and block PD-1 receptors on T-cells without causing inhibitor signaling transduction. Such peptides include B7-DC polypeptide, B7-H1 polypeptide, B7-1 polypeptide and B7-2 polypeptide and soluble fragments thereof, as disclosed in U.S. Patent No. 8,114,845.

Another class of immune checkpoint inhibitors includes compounds having peptide moieties that inhibit PD-1 signaling. Examples of such compounds are disclosed in U.S. Patent No. 8,907,053.

Another class of immune checkpoint inhibitors includes inhibitors of certain metabolic enzymes, such as indoleamine 2,3-dioxygenase (IDO), which is expressed by infiltrating bone marrow cells and tumor cells. IDO enzymes suppress immune responses, either by depleting T cells of amino acids needed for anabolism or by synthesizing specific natural ligands for cytoplasmic receptors that can alter lymphocyte function.

The compositions of the invention (e.g., polypeptides, polynucleotides, etc.) can be generally formulated in a pharmaceutically-acceptable or physiologically-acceptable solution, alone or in combination with one or more other therapeutic modalities, for administration to cells, tissues, or animals. If desired (as needed), the compositions of the present invention may be administered in combination with other agents (e.g., other proteins, polypeptides, or various pharmaceutically active agents). There is virtually no limit to the other ingredients that may be included in the compositions of the present invention, provided that the additional agents do not adversely affect the properties of the peptides and the like of the present invention.

Formulations of pharmaceutically acceptable excipients and carriers for the compositions of the present invention are known to those skilled in the art. In addition, appropriate dosages and therapeutic regimens for the use of certain compositions described herein may be employed in any manner well known to those skilled in the art, including, for example, oral, parenteral, intravenous, intranasal, intracerebral, and intramuscular administration and formulations therefor.

In certain applications, the pharmaceutical compositions disclosed herein can be delivered to a subject by oral administration. As such, the compositions may be formulated with an inert diluent or with an absorbable edible carrier, or the composition may be enclosed in a hard-shell or soft-shell gelatin capsule, or the compositions may be compressed into tablets, or the compositions may be directly incorporated into a food product.

In certain circumstances, the pharmaceutical compositions disclosed herein may be preferably delivered parenterally, intravenously, intramuscularly, or intraperitoneally, and such routes of administration are described in, for example, U.S. Patent No. 5,543,158; U.S. Patent No. 5,641,515; U.S. Patent No. 5,399,363, and others, which are hereby incorporated by reference. A solution of the active compound (as a free base or as a pharmaceutically acceptable salt) can be prepared by mixing it with a surfactant, such as hydroxypropylcellulose, in water, as appropriate. The dispersion may also be prepared in glycerol, liquid polyethylene glycol, or mixtures thereof, or may be prepared in oil. The formulations may include a preservative to prevent microbial growth under normal conditions of storage and use.

Pharmaceutical forms suitable for injectable use include sterile aqueous solutions or dispersions, and sterile powders that allow for the extemporaneous preparation of sterile injection solutions or dispersions (U.S. Patent No. 5,466,468 is hereby incorporated by reference). In all cases, the pharmaceutical form must be sterile and flowable enough to facilitate injection. It must be stable under production and storage conditions and must be preservative against the contaminating action of microorganisms such as bacteria and fungi. The carrier can be a solvent or dispersion medium, for example, water, ethanol, polyols (e.g., glycerol, propylene glycol, and liquid polyethylene glycol), suitable mixtures thereof, and/or vegetable oils. Suitable fluidity can be maintained, for example, by using a coating agent such as lecithin, by maintaining the required particle size for dispersion, and by using a surfactant. Prevention of microbial action can be achieved by various antibacterial and antifungal agents (e.g., parabens, chlorobutanol, phenols, sorbic acid, thimerosal, etc.). In many cases, it is desirable to include an isotonic agent, such as sugar or sodium chloride. Prolonged absorption of the injectable composition may be achieved by incorporating into the composition agents that delay absorption (e.g., aluminum monostearate and gelatin).

For parenteral administration of the aqueous solution, for example, the solution should be appropriately buffered as necessary, and the liquid diluent should first be isotonicized with sufficient physiological saline or glucose. These particular aqueous solutions are particularly suitable for intravenous administration, intramuscular administration, subcutaneous administration and intraperitoneal administration. Sterile aqueous media that may be used in this regard are known to those skilled in the art. For example, a single dose may be dissolved in 1 ml of an isotonic NaCl solution, added to 1000 ml of hypodermoclysis fluid, or injected into the proposed injection site. Some variation in dosage will necessarily occur depending on the symptoms of the subject being treated with the pharmaceutical composition. One of ordinary skill in the art will be able to determine the appropriate dose for an individual subject. In addition, for administration to humans, the compositions must meet the sterility, pyrogenicity, and general safety and purity standards required by the FDA Office of Biologics standards.

Sterile injection solutions may be prepared by including the active compound in the required amount in a suitable solvent, together with various other components listed above as needed, followed by filtration sterilization. In general, dispersions may be prepared by including the various sterile active ingredients in a sterile vehicle comprising a base dispersion medium and the various other necessary components listed above. In the case of sterile powders for the preparation of sterile injection solutions, preferred methods of preparation may be vacuum-drying and freeze-drying techniques, which produce a powder of the active ingredient plus any additional desired components from a previously sterilized and filtered solution.

The compositions disclosed herein may be formulated in neutral or salt form. Pharmaceutically-acceptable salts may include acid-adduct salts (formed with free amino groups of the protein), wherein the acid-adduct salt is formed with an inorganic acid (e.g., hydrochloric acid or phosphoric acid, etc.) or an organic acid (acetic acid, oxalic acid, tartaric acid, mandelic acid, etc.). Salts formed with free carboxyl groups can also be derived from inorganic bases (e.g., sodium hydroxide, potassium hydroxide, ammonium hydroxide, calcium hydroxide, or iron hydroxide) and organic bases (e.g., isopropylamine, trimethylamine, histidine, procaine, etc.). Depending on the formulation, the solution is administered in a manner appropriate to the dosage form and in a therapeutically effective amount. The formulations can be readily administered in a variety of dosage forms, such as, for example, injection solutions, drug-releasing capsules, and the like.

As used herein, the term 'carrier' includes any solvent, dispersion medium, vehicle, coating, diluent, antibacterial and antifungal agent, isotonic and absorption retarder, buffer, carrier solution, suspension, colloid, and the like. The use of such media and agents for pharmaceutically active substances is well known in the art. They are used in therapeutic compositions, except where any conventional medium or agent is incompatible with the active ingredient. Auxiliary active ingredients may also be included in the compositions of the present invention.

The expression 'pharmaceutically acceptable' refers to molecular entities and compositions that, when administered to humans, do not cause allergic or similar adverse reactions. Methods of preparing aqueous compositions comprising proteins as active ingredients are well known in the art. Typically, such compositions are prepared as injectable liquid solutions or suspensions; solid forms suitable for dissolution or suspension in a liquid prior to injection may also be prepared. Additionally, the compositions may be emulsified.

In certain embodiments, pharmaceutical compositions can be delivered by intranasal spray, inhalation, and/or other aerosol delivery vehicles. Methods for delivering gene, polynucleotide, and peptide compositions directly to the lungs via nasal aerosol sprays are described in, for example, U.S. Patent No. 5,756,353 and U.S. Patent No. 5,804,212, which are hereby incorporated by reference. Similarly, intranasal delivery of drugs using microparticulate resins (Takenaga et al, 1998) and rhizophosphatidyl-glycerol compounds (U.S. Patent No. 5,725,871) are also well known in the pharmaceutical field. Similarly, transmucosal drug delivery in the form of polytetrafluoroetheylene support matrices is described in U.S. Patent No. 5,780,045, which is hereby incorporated by reference.

In certain embodiments, the delivery can be accomplished by utilizing liposomes, nanocapsules, microparticles, microspheres, lipid particles, vesicles, and the like for introducing the compositions of the invention into appropriate cells. In particular, the compositions of the present invention can be encapsulated in lipid particles, liposomes, vesicles, nanospheres, or nanoparticles and formulated for delivery. The formulation and utilization of these delivery vehicles can be performed using known prior art techniques.

In addition, the pharmaceutical compositions of the present invention can be formulated using methods known in the art to provide a rapid, sustained, or delayed release of the active ingredient after administration to a mammal. A pharmaceutical composition formulated in such a manner can be administered in an effective amount, as described above, by any of several routes, including orally, transdermally, subcutaneously, intravenously, or intramuscularly. As used herein, an `effective amount' is an amount of a substance that, when administered to a patient, allows for the detection of a diagnostic or therapeutic effect. A pharmaceutical composition comprising a polypeptide of the present invention may be administered several times daily at an effective amount of 0.1 µg to 10,000 mg per dose, preferably 1 mg to 5,000 mg, based on an adult, although the amount of active ingredient may vary depending on the severity of the disease. However, the dosage of the pharmaceutical composition according to the present invention may be appropriately selected depending on the route of administration, the subject to whom it is administered, the subject's disease and its severity, age, sex body weight, individual differences and disease state, and such techniques are known to those skilled in the art.

In another aspect, the present invention provides methods of utilizing a composition (e.g., polynucleotide, polypeptide, etc.) of the present invention in a cell, tissue, or subject to achieve a desired cellular effect and/or therapeutic effect. The cells or tissues that may be modulated by the present invention may preferably be mammalian cells or tissues, and more preferably human cells or tissues. Such cells or tissues may be in a healthy state or in a diseased state.

The present invention also provides a pharmaceutical composition for preventing or treating cancer comprising at least one selected from the group consisting of the following (i) to (iv):
(i) the peptide,
(ii) a polynucleotide encoding (i) the peptide;
(iii) a vector comprising (ii) the polynucleotide, and
(iv) a host cell transformed by (iii) the vector.

According to one aspect of the present invention, a peptide comprising the amino acid sequence of SEQ ID NO: 2; or a peptide comprising an amino acid sequence exhibiting at least 95% homology to said peptide, can both exert an inhibitory and killing effect on tumor growth by itself and thus have a preventive or therapeutic effect on cancer.

The present invention also provides use of the at least one selected from the group consisting of the following (i) to (iv) for preparation of an agent for the treatment of cancer:
(i) the peptide,
(ii) a polynucleotide encoding (i) the peptide;
(iii) a vector comprising (ii) the polynucleotide, and
(iv) a host cell transformed by (iii) the vector.

The present invention also provides a method for treating cancer comprising administering to a subject in need thereof an effective amount of a composition comprising at least one selected from the group consisting of the following (i) to (iv):
(i) the peptide,
(ii) a polynucleotide encoding (i) the peptide;
(iii) a vector comprising (ii) the polynucleotide, and
(iv) a host cell transformed by (iii) the vector.

The term `effective amount' of the present invention means an amount that exhibits an effect of improving, treating, preventing, detecting, diagnosing, or suppressing or reducing cancer when administered to a subject. The 'subject' may be animals, preferably, mammals, particularly animals including humans and may also be cells, tissues, and organs derived from animals. The subject may be a patient requiring the effects.

The term 'treatment' of the present invention comprehensively refers to improving cancer or symptoms of cancer, and may include treating or substantially preventing these diseases or improving the conditions thereof and includes alleviating, treating or preventing a symptom or most of symptoms derived from the diseases, but is not limited thereto.

### ADVANTAGEOUS EFFECT

The peptide disclosed in the present invention is a CRS fragment disclosed for the first time in the present specification and exhibits anticancer activity and immune function enhancing activity.

In addition, the peptide, a polynucleotide encoding the same, a vector comprising the polynucleotide, a host cell transformed with the vector, or a CRS full-length protein has excellent anticancer activity and immune function enhancing activity, so it can be very usefully used in the development of vaccine adjuvants, vaccine compositions, and cancer treatment compositions.

### BRIEF DESCRIPTION OF THE DRAWINGS

FIG. 1 is a schematic diagram briefly illustrating a C-VAX development method.
FIG. 2 is the result of confirming the stability of the protein by purifying the intermediates derived from the C-VAX development process, proceeding with SDS-PAGE using acrylamide gel, and then staining the protein with Coomasiae blue dye.
FIG. 3 is the result of confirming whether UNE-C1-4H and C-VAX are multimer through size exclusion chromatography and FPLC.
FIG. 4 is the result of confirming the TNF-α level in the medium through ELISA by treating the developed C-VAX treated with his-UNE-C1-his and PMA (50 ng/ml) for 48 hours, then treating with differentiated THP-1 cells for 4 hours.
FIGs. 5 and 6 are the results of confirming IL-6(e), IL-12 p70 (f) present in the medium through ELISA after treating BMDC with his-UNE-C1-his and C-VAX at each concentration for 24 hours, respectively.
FIG. 7 is an experimental result of confirming the immune activity of each cell line by treating each HEK-Blue cell line with his-UNE-C-his, C-VAX at each concentration for 24 hours, and then collecting the supernatant and reacting with the QUANTI-Blue solution.
FIG. 8 is the results of observing the size of tumors until Day 17 after Eg7-OVA cells (5×10⁵) were transplanted subcutaneously on the right back of c57bl/6 mice, then treated with OVA(10ug/mouse), or OVA(10ug/mouse) + his-UNE-C1-his (100ug/mouse) or OVA (10ug/mouse) + C-VAX (100ug/mouse) on Days 3 and 10.

### MODE FOR CARRYING OUT INVENTION

Hereinafter, the present invention will be described in detail.

However, the following examples are only to illustrate the present invention, and the content of the present invention is not limited to the following examples.

### Experiment method

### 1. Mouse

C57BL/6 and BALB/c mice were obtained from Duyul Biotech. In addition, OVA-specific T cell receptor transgenic OT-1 mice were kindly provided by Professor Kang Chang-yul of Seoul National University. All mouse experiments were performed in accordance with guidelines approved by Seoul National University. All mice were maintained on a Woojung bsc using institutionally approved protocols.

### 2. Cell lines

CT26 and B16f10 CRS overexpressing cells were generated by the G418 selection method. Briefly, lipofectamine 2000 was used to transform pEXPR-IBA105 EV and CRS into each cell. To select overexpressed cells, 1 mg/ml of G418 was used in the growth medium and resistant single clones were selected. The expression level of each clone was tested by immunoblotting, and clones with similar in vitro growth were selected and used in the experiment.

### 3. Allogeneic Mouse Tumor Models and Tumor Measurements

CT26 and B16F10 were maintained in DMEM containing 10% FBS and 1% streptomycin. A total of 5 × 10⁵ cells of CT26 and B16F10 were subcutaneously injected into the right side of BALB/c and C57BL/6 mice aged 6-8 weeks, respectively. On days 7, 8 and 9, 200 µg of each protein was injected intraperitoneally. Tumors were measured with digital calipers and calculated according to the following equation (0.52 X length X width²). Mice were euthanized when tumors reached more than 1500 mm³.

### 4. Cell binding Assay

After separating the spleens of C57BI/6 mice, physical force is applied to isolate and divide them into single cells. It is incubated for 1 hour at 4 °C with splenocytes divided using BSA and CRS protein stained with Alexa647 dye. The cultured cells were stained with CD11b, CD11c, CD3, CD19, Ly6C, Ly6G, and F4/80 FACS antibody, and analyzed through FACS analysis. CD3 : T cell, CD11b+, F4/80+ : macrophage, CD11b, Ly6C : monocyte, CD11b+, Ly6G :neutrophil, CD19 : B cell, CD11b+, CD11c+ : Dendritic cell

### 5. Tumor-infiltrating immune cell and splenic immune cell assays

CT26 tumor-bearing mice were sacrificed on days 10 and 12. Tumor cells were dissociated using a Tumor dissociation kit, mouse (130-096-730, Miltenyi Biotec) according to the manufacturer's protocol. Splenocytes were dissociated in medium containing RPMI medium containing 2% FBS and 1% streptomycin. After lysis of red blood cells (Lysing Buffer (555899, BD biosciences)), tumor and spleen cells were stained with CD8, CD4, CD3, CD45, Foxp3 and CD69 antibodies. Monoclonal antibodies were stained for 30 minutes at 4°C. For intracellular staining, a Fixation/Permeabilization Solution Kit with BD GolgiPlug^{™} (555028, BD biosciences) was used.

### 6. Protein Purification

CRS and CRS (106-228) proteins were purified using the pET28a plasmid vector containing N and C-terminal 6X his tags. In the case of His-UNE-C1-4H and C-VAX, purification was performed using a pHIS.Parallell plasmid vector containing an N-terminal 6X his tag and a region cleavable by rTEV. Transformed into BL21-codon plus cells and colonies were inoculated into medium and grown. Large scale cells were grown in LB until OD 600 reached 0.5 and protein expression was induced using 0.5 mM IPTG for 16 hours at 4°C. A cell pellet was obtained from centrifugation and disrupted by sonication in 50 mM Tris buffer pH7.5 containing 300 mM NaCl. Then, the supernatant was obtained by centrifugation at 20,000 g for 30 min. It was poured over a column containing Ni-NTA resin. Wash steps were performed with 50 mM Tris, pH 7.5 containing 300 mM NaCl, 5% glycerol and 15 mM imidazole. Proteins were separated from the column with 10 ml of elution buffer (50 mM Tris pH7.5, 300 mM NaCl, 5% glycerol, 300 mM imidazole). In the case of His-UNE-C1-4H and C-VAX, rTEV protease was mixed at 1:20 (g) of the total amount of target protein, then dialysis was performed and endotoxin was removed using TX-114 (REF: Removal of endotoxin from protein solutions by phase separation using Triton X-114). After filling the poly-prep column with SM-2 beads, the remaining triton x-114 was removed by passing through the protein according to the manufacturer's recommended protocol. Titrated protein of 0.04 EU/mg or less from the LAL assay was used for the entire experiment.

### 7. ELISA

THP1-PMA and BMDC (bone marrow derived DC) cells were tested to confirm cytokine secretion. It was treated with 5 X 10⁵ cell/ml in a 24-well plate, and each well was changed to a serum-free medium for 2 hours before drug treatment. In the case of THP1-PMA, 100 nM of protein was treated for 4 hours. For BMDC cells, 100 nM concentration of protein was treated for 24 hours. ELISA was performed using the IL-6, TNF-α and IL-12 ELISA set (BD) by centrifuging the supernatant at 500g for 10 minutes.

### 8. Activate BMDC and BMM

Bone marrow derived dendritic cells (BMDC) were prepared using standard methods. Briefly, bone marrow cells were obtained from female C57BL/6 mice and cultured in RPMI medium containing 10% FBS, 1% streptomycin and 10ng/ml GM-CSF (R&D, BMDC) or 10ng/ml M-CSF (R&D, BMM). On day 3, GM-CSF or M-CSF and fresh RPMI medium were added and BMDC were harvested from non-adherent and loosely adherent cells on day 6 and BMM from adherent cells. 100 nM of each protein and 1 µg/ml of LPS were treated for 18 hours, followed by FACS analysis using antibodies against CD11c, CD40, CD80 and CD86.

### 9. Therapeutic anticancer vaccine mouse model

In a therapeutic cancer vaccine model using C-VAX, E.G7-OVAcells were injected s.c into the right side of the back. On days 3 and 10, OVA (10ug/mouse), OVA (10ug/mouse) + his-UNE-C1-his (100ug/mouse), OVA (10ug/mouse) + C-VAX (100ug/mouse) were injected into the left side of the back respectively, tumor volume was measured as mentioned above.

### 10. Size Exclusion Chromatography (FPLC: Fast Protein Liquid Chromatography)

Protein samples were concentrated to the required concentration using amicon Ultra-15 centrifugal fiter units and a buffer consisting of 50 mM Tris, pH 7.5, 300 mM NaCl. After connecting the column (GE healthcare, Chicgo, Illinois, USA) with AKTA pure (GE Healthcare), it was washed with a buffer three times the volume of the column, and equilibrated with the protein buffer. After equilibration was made, the protein sample was injected into AKTA pure, and the experiment was performed according to the protocol according to the execution conditions according to the manufacturer's instructions.

### 11. HEK-blue SEAP assay

HEK cells were cultured in DMEM containing 1% antibiotics, 10% FBS, and 100ug/ml Normocin (Invivogen, San Diego, CA, USA) in an incubator at 37 °C under 5% CO₂. After processing the protein to be processed in a 24 well plate, 5×10⁵ cells of hTLR2, hTLR1/TLR2 and TLR2KO-hTLR1/TLR2-HEK-Blue cells were added to each well. Plates were incubated for 24 hours and supernatants were collected. The collected supernatant was mixed with QUANTI-Blue solution (InvivoGen), incubated at 37 °C for 15 minutes to 6 hours, and the result was measured at OD 620nm.

### Experiment result

### 1. Development of C-VAX, a form of drug development, and verification of immune activity and anticancer efficacy

After confirming the efficacy of CRS (106-228) in an existing study, the present inventors conducted research to make CRS (106-228) a form capable of drug development. A form that can be developed as a drug must satisfy three conditions: (1) no tag, (2) stability without protein degradation, and (3) the same or higher potency than the initial form.

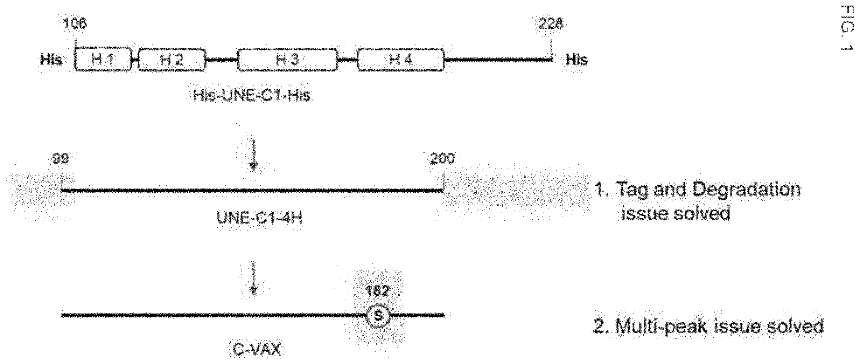

The CRS (106-228) used in previous studies has his tags attached to both ends of the protein, and in the present invention, the CRS (106-228) fragment was named his-UNE-C1-his. In the present invention, a study was conducted to remove the his-tags at both ends of the his-UNE-C1-his and convert the multi-form into a single form by cysteine (FIG. 1).

### (1) Removal of His tag and control of protein sequence (production of UNE-C1-4H)

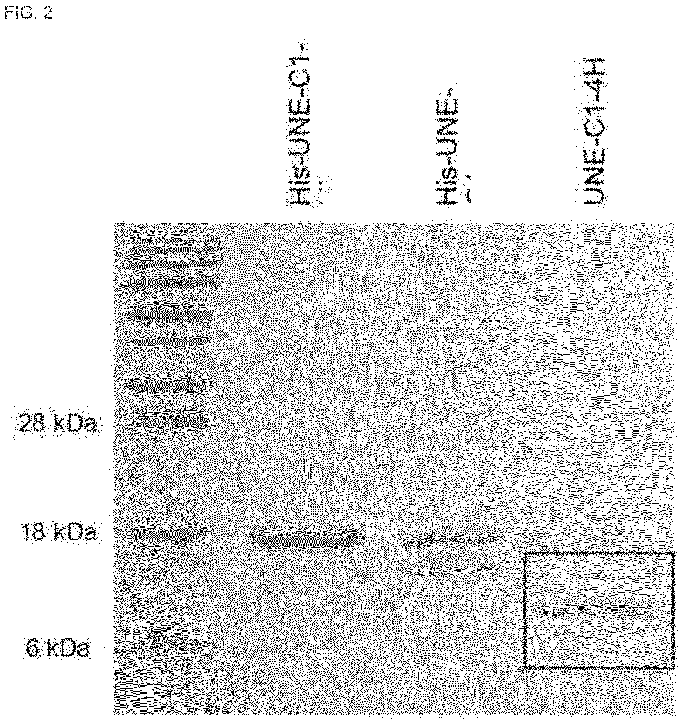

In the His-UNE-C1-his protein, the C-terminal his tag was first removed (his-UNE-C1), and then protein degradation was confirmed. To stabilize this, the protein sequence was adjusted from 106-228aa to 99-200aa of CRS (SEQ ID NO: 1), the sequence-controlled CRS (99-200aa) peptide (hereinafter referred to as 'UNE-C1-4H') was confirmed to be in a stable form without protein degradation (FIG. 2).

### (2) Manufacture of peptides in single form without multimer form

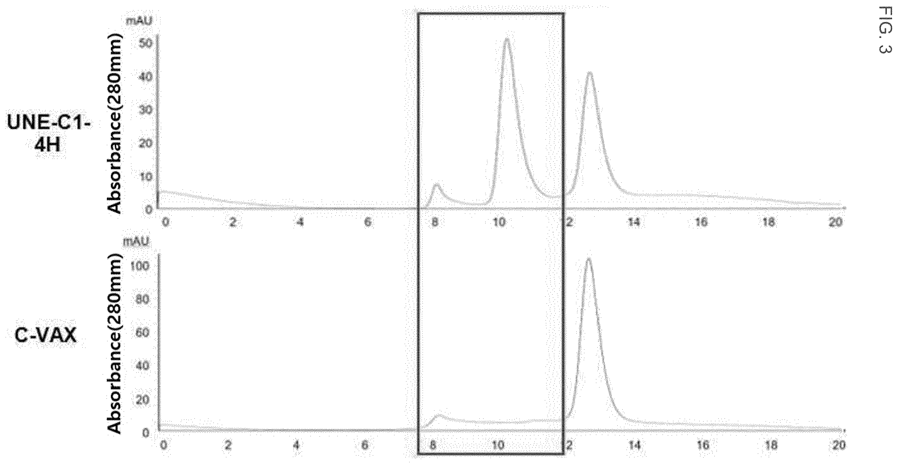

It was confirmed that the prepared UNE-C1-4H exists in two forms, a trimer and a monomer, through FPLC (Fast protein liquid chromatography). To solve this problem, the cysteine residue present in UNE-C1-4H was modified to a serine residue, and it was confirmed that only the monomer was present when confirmed through FPLC (FIG. 3). UNE-C1-4H in which the cysteine residue was modified into a serine residue was named C-VAX.

### (3) Immunoactivity of C-VAX

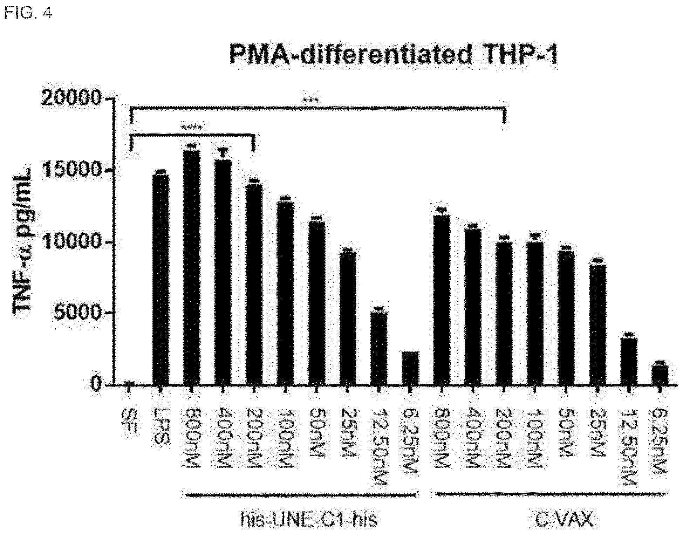

Using the PMA-differentiated THP-1 cell line, the macrophage immune activation efficacy of his-UNE-C1-his and C-VAX was confirmed. As a result of the experiment, it was confirmed that both C-VAX and his-UNE-C1-his showed sufficient immune activation efficacy (FIG. 4).

In addition, the immune activation efficacy of his-UNE-C1-his and C-VAX was confirmed using bone marrow-derived dendritic cells (BMDC). As a result of the experiment, it was confirmed that both C-VAX and his-UNE-C1-his increased IL-6 and IL-12p70 cytokine secretion in BMDC (FIGS. 5 and 6).

### (4) C-VAX immune activation mechanism through TLR2/6

Since previous studies have confirmed that His-UNE-C1-his causes immune activity through TLR2/6, in the present invention, we tried to confirm whether C-VAX made based on his-UNE-C1-his also has immune activity through TLR2/6.

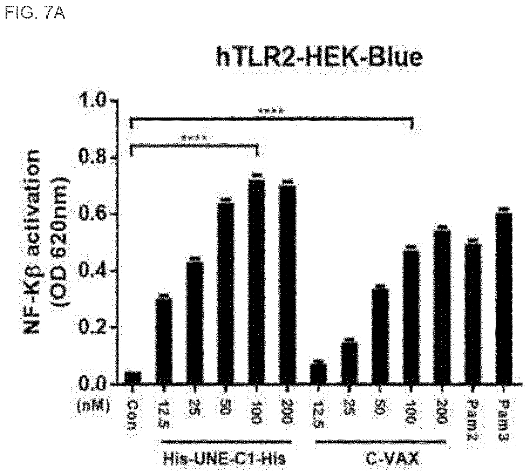

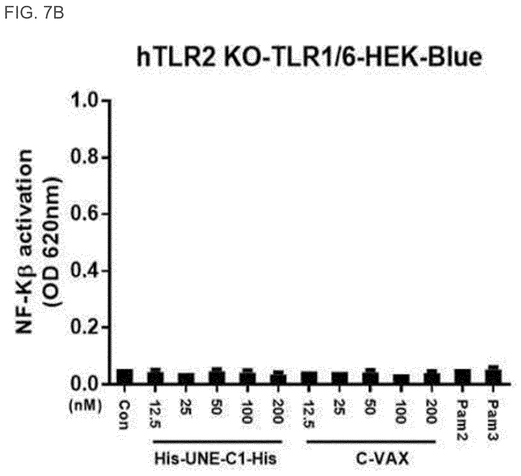

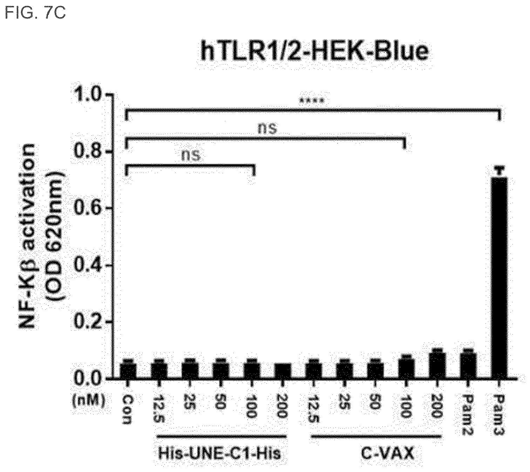

As a result of experiments using hTLR2-HEK-blue cell line, hTLR2 KO-TLR1/6-HEK-Blue cell line, and hTLR1/2-HEK-blue cell line, His-UNE-C1-his and C-VAX exhibited immunoactivity in the same pattern, and TLR2 was very important, and it has been confirmed that TLR1/6 are ineffective. Therefore, it was found that C-VAX also uses TLR2/6 as a receptor, and C-VAX exhibits immunoactivity by the same mechanism as his-UNE-C1-his (FIG. 7).

### (5) Anticancer efficacy of C-VAX

Since the efficacy of his-UNE-C1-his as an anti-cancer vaccine was confirmed in a previous study, the anti-cancer vaccine efficacy of C-VAX was also confirmed.

Right subcutaneous injection of the E.G7-OVA cell line with the number of 5×10⁵ cells, and left subcutaneous injection of his-UNE-C1-his, C-VAX 5mpk, and OVA protein was performed on day-3 and day-7, and the size of the cancer and the weight of the mouse were measured.

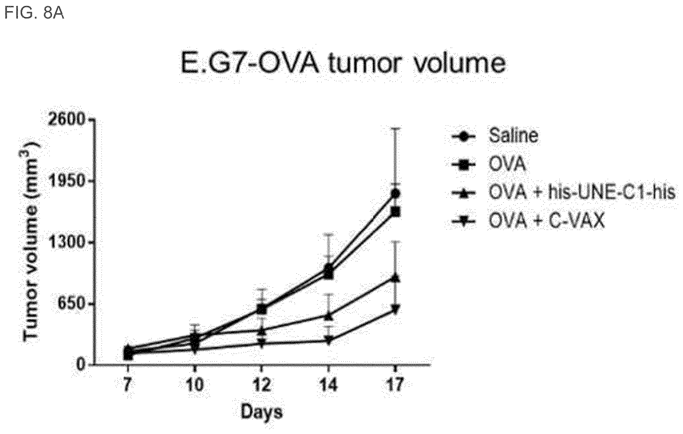

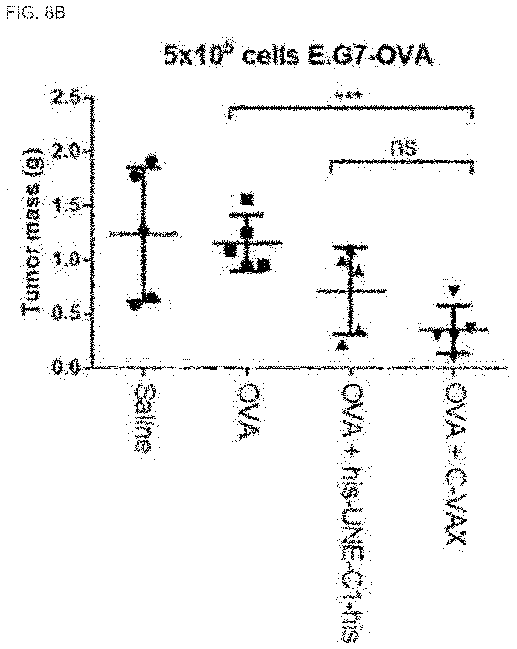

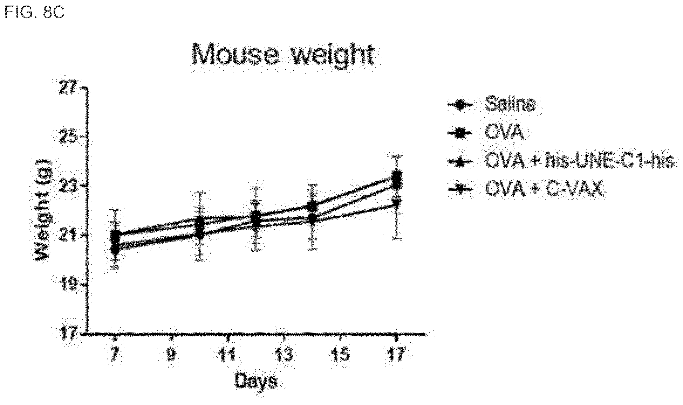

When C-VAX was treated with OVA, it was confirmed that a strong anticancer effect was shown, as was the case when his-UNE-C1-his was treated with OVA (FIG. 8).

### INDUSTRIAL APPLICABILITY

The peptide disclosed in the present invention is a CRS fragment disclosed for the first time in the present specification and exhibits anticancer activity and immune function enhancing activity.

In addition, the peptide, a polynucleotide encoding the same, a vector comprising the polynucleotide, a host cell transformed with the vector, or a CRS full-length protein has excellent anticancer activity and immune function enhancing activity, so it can be used very usefully in the development of vaccine adjuvants, vaccine compositions, and cancer treatment compositions, and thus has excellent industrial applicability.

## Claims

1. A peptide consisting of the amino acid sequence of SEQ ID NO: 2 or an amino acid sequence showing 95% or more sequence homology thereto.

2. The peptide according to claim 1, wherein the peptide activates innate immunity and adaptive immunity.

3. The peptide according to claim 1, wherein the peptide consists of the amino acid sequence of SEQ ID NO: 2 or SEQ ID NO: 3.

4. A polynucleotide comprising a nucleotide sequence encoding the peptide of claim 1.

5. The polynucleotide according to claim 4, wherein the polynucleotide consists of the nucleotide sequence of SEQ ID NO: 4.

6. A vector comprising the polynucleotide of claim 5.

7. A host cell transformed with the vector of claim 6.

8. A vaccine adjuvant comprising at least one selected from the group consisting of the following (i) to (iv).
(i) the peptide of claim 1,
(ii) a polynucleotide encoding (i) the peptide;
(iii) a vector comprising (ii) the polynucleotide, and
(iv) a host cell transformed by (iii) the vector.

9. A vaccine composition comprising the vaccine adjuvant of claim 8 and an antigen.

10. The vaccine composition according to claim 9, wherein the antigen is at least one selected from the group consisting of alpha-fetoprotein, carcinoembryonic antigen, cdk4, beta-catenin, CA125, caspase-8, epithelial tumor antigen, HPV antigen, HPV16 antigen, CTL epitope derived from HPV16 E7 antigen, melanoma associated antigen (MAGE)-1, MAGE-3, tyrosinase , surface Ig idiotype, Her-2/neu, MUC-1, prostate specific antigen (PSA), sialyl Tn (STn), heat shock protein, gp96, ganglioside molecules GM2, GD2, GD3, carcinoembryonic antigen (CEA), PRAME, WT1, survivin, cyclin D, cyclin E, HER2, MAGE, NY-ESO, EGF, GP100, cathepsin G, human papillomavirus (HPV)-16- E6, HPV-16-E7, HPV-18-E6, HPV-18-E7, Her/2-neu antigen, chimeric Her2 antigen, prostate specific antigen (PSA), bivalent PSA, ERG, androgen receptor (AR), PAK6, prostate stem cell antigen (PSCA), NY-ESO-1, Stratum Corneum Chymotryptic Enzyme (SCCE) antigen, Wilms tumor antigen 1 (WT-1), HIV-1 Gag, Human Telomerase Reverse Transcriptase (hTERT), proteinase 3, tyrosinase-related protein 2 (TRP2), high molecular weight melanoma-associated antigen (HMW-MAA), synovial sarcoma, X (SSX)-2, carcinoembryonic antigen (CEA), melanoma-associated antigen E (MAGE-A, MAGE1, MAGE2, MAGE3, MAGE4), interleukin-13 receptor alpha (IL13-R alpha), carbonic anhydrase IX (CAIX), survivin, GP100, angiogenic antigen, ras protein, p53 protein, p97 melanoma antigen, KLH antigen, carcinoembryonic antigen (CEA), gp100, MART1 antigen, TRP-2, HSP-70, beta-HCG, testicine, 1A01_HLA-A/m; 1A02; 5T4; ACRBP; AFP; AKAP4; alpha-actinin-_4/m; alpha-methylacyl-coenzyme_A_racemase; ANDR; ART-4; ARTC1/m; AURKB; B2MG; B3GN5; B4GN1; B7H4; BAGE-1; BASI; BCL-2; bcr/abl; beta-catenin/m; BING-4; BIRC7; BRCA1/m; BY55; calreticulin; CAMEL; CASPA; caspase_8; cathepsin_B; cathepsin_L; CD1A; CD1B; CD1C; CD1D; CD1E; CD20; CD22; CD276; CD33; CD3E; CD3Z; CD4; CD44_isoform_1; CD44_isoform_6; CD52; CD55; CD56; CD80; CD86; CD8A; CDC27/m; CDE30; CDK4/m; CDKN2A/m; CEA; CEAM6; CH3L2; CLCA2; CML28; CML66; COA-1/m; coactosin-like_protein; collagen_XXIII; COX-2; CP1B1; CSAG2; CT-_9/BRD6; CT45A1; CT55; CTAG2_isoform_LAGE-1A; CTAG2_isoform_LAGE-1B; CTCFL; Cten; cyclin_B1; cyclin_D1; cyp-B; DAM-10; DEP1A; E7; EF1A2; EFTUD2/m; EGFR; EGLN3; ELF2/m; EMMPRIN; EpCam; EphA2; EphA3; ErbB3; ERBB4; ERG; ETV6; EWS; EZH2; FABP7; FCGR3A_version_1; FCGR3A_version_2; FGF5; FGFR2; fibronectin; FOS; FOXP3; FUT1; G250; GAGE-1; GAGE-2; GAGE-3; GAGE-4; GAGE-5; GAGE-6; GAGE7b; GAGE-8_ (GAGE-2D); GASR; GnT-V; GPC3; GPNMB/m; GRM3; HAGE; hepsin; Her2/neu; HLA-A2/m; Homeobox_NKX3.1; HOM-TES-85; HPG1; HS71A; HS71B; HST-2; hTERT; iCE; IF2B3; IL-10; IL-13Ra2; IL2-RA; IL2-RB; IL2-RG; IL-5; IMP3; ITA5; ITB1; ITB6; kallikrein-2; kallikrein-4; KI20A; KIAA0205; KIF2C; KK-LC-1; LDLR; LGMN; LIRB2; LY6K; MAGA5; MAGA8; MAGAB; MAGE-_B1; MAGE-_E1; MAGE-A1; MAGE-A10; MAGE-A12; MAGE-A2; MAGE-A3; MAGE-A4; MAGE-A6; MAGE-A9; MAGE-B10; MAGE-B16; MAGE-B17; MAGE-B2; MAGE-B3; MAGE-B4; MAGE-B5; MAGE-B6; MAGE-C1; MAGE-C2; MAGE-C3; MAGE-D1; MAGE-D2; MAGE-D4; MAGE-E1_(MAGE1); MAGE-E2; MAGE-F1; MAGE-H1; MAGEL2; mammaglobin_A; MART-1/melan-A; MART-2; MC1_R; M-CSF; mesothelin; MITF; MMP1_1; MMP7; MUC-1; MUM-1/m; MUM-2/m; MYO1A; MYO1B; MYO1C; MYO1D; MYO1E; MYO1F; MYO1G; MYO1H; NA17; NA88-A; Neo-PAP; NFYC/m; NGEP; N-myc; NPM; NRCAMs; NSE; NUF2; NY-ESO-1; OA1; OGT; OS-9; osteocalcin; osteopontin; p53; PAGE-4; PAI-1; PAI-2; PAP; PATE; PAX3; PAX5; PD1L1; PDCD1; PDEF; PECA1; PGCB; PGFRB; Pim-1_-kinase; Pin-1; PLAC1; PMEL; PML; POTE; POTEF; PRAME; PRDX5/m; PRM2; prostein; proteinase-3; PSA; PSB9; PSCA; PSGR; PSM; PTPRC; RAB8A; RAGE-1; RARA; RASH; RASK; RASN; RGS5; RHAMM/CD168; RHOC; RSSA; RU1; RU2; RUNX1; S-100; SAGE; SART-1; SART-2; SART-3; SEPR; SERPINB5; SIA7F; SIA8A; SIAT9; SIRT2/m; SOX10; SP17; SPNXA; SPXN3; SSX-1; SSX-2; SSX3; SSX-4; ST1A1; STAG2; STAMP-1; STEAP-1; survivin; survivin-2B; SYCP1; SYT-SSX-1; SYT-SSX-2; TARP; TCRg; TF2AA; TGFbeta1; TGFR2; TGM-4; TIE2; TKTL1; TPI/m; TRGV11; TRGV9; TRPC1; TRP-p8; TSG10; TSPY1; TVC_(TRGV3); TX101; tyrosinase; TYRP1; TYRP2; UPA; VEGFR1; WT1; XAGE1, alpha-actinin-4; ARTC1; BCR-ABL fusion protein (b3a2); B-RAF; CASP-5; CASP-8; beta-catenin; Cdc27; CDK4; CDKN2A; COA-1; dek-can fusion protein; EFTUD2; Elongation factor 2; ETV6-AML1 fusion protein; FN1; GPNMB; LDLR-fucosyltransferase AS fusion protein; HLA-A2d; HLA-A11d; hsp70-2; KIAAO205; MART2; ME1; MUM-If; MUM-2; MUM-3; neo-PAP; myosin class I; NFYC; OGT; OS-9; pml-RARalpha fusion protein; PRDX5; PTPRK; K-ras; N-ras; RBAF600; SIRT2; SNRPD1; SYT-SSX1 or SSX2 fusion protein; Triosephosphate Isomerase; BAGE-1; GAGE-1,2,8; GAGE-3,4,5,6,7; GnTVf; HERV-K-MEL; KK-LC-1; KM-HN-1; LAGE-1; MAGE-A1; MAGE-A2; MAGE-A3; MAGE-A4; MAGE-A6; MAGE-A9; MAGE-A10; MAGE-A12; MAGE-C2; mucin k; NA-88; NY-ESO-1/LAGE-2; SAGE; Sp17; SSX-2; SSX-4; TRAG-3; TRP2-INT2g; CEA; gp100/Pmel17; Kallikrein 4; mammaglobin-A; Melan-A / MART-1; NY-BR-1; OA1; PSA; RAB38/NY-MEL-1; TRP-1/gp75; TRP-2; tyrosinase; adipophilin; AIM-2; BING-4; CPSF; cyclin D1; Ep-CAM; EphA3; FGF5; G250/MN/CAIX; HER-2/neu; IL13Ralpha2; Intestinal carboxyl esterase; Alpha-foetoprotein; M-CSF; mdm-2; MMP-2; MUC1; p53; PBF; PRAME; PSMA; RAGE-1; RNF43; RU2AS; secernin 1; SOX10; STEAP1; survivin; telomerase; WT1; FLT3-ITD; BCLX(L); DKK1; ENAH (hMena); MCSP; RGS5; gastrin-17; Human Chorionic Gonadotropin, EGFRvIII, HER2, HER2/neu, P501, Guanylyl Cyclase C, prostatic acid phosphatase (PAP), ovalbumin (OVA) and MART-1.

11. The vaccine composition according to claim 9 or 10, wherein the vaccine is an anti-cancer vaccine.

12. The vaccine composition according to claim 11, wherein the anti-cancer vaccine is a vaccine for preventing cancer or a vaccine for treating cancer.

13. The vaccine composition according to claim 11, wherein the cancer is at least one selected from the group consisting of breast cancer, colorectal cancer, prostate cancer, cervical cancer, stomach cancer, skin cancer, head and neck cancer, lung cancer, glioblastoma, oral cancer, pituitary adenoma, glioma, brain tumor, pharyngeal cancer, laryngeal cancer, thymoma, mesothelioma, esophageal cancer, rectal cancer, liver cancer, pancreatic cancer, pancreas endocrine tumors, gallbladder cancer, penile cancer, ureter cancer, renal cell cancer, bladder cancer, non-Hodgkin's lymphoma, myelodysplastic syndrome, multiple myeloma, plasma cell tumor, leukemia, childhood cancer, bronchial cancer, colon and ovarian cancer.

14. The vaccine composition according to claim 11, further comprising at least one selected from the group consisting of vaccine adjuvants, immune checkpoint inhibitors, and combinations thereof.

15. The vaccine composition according to claim 14, wherein the vaccine adjuvant is at least one selected from the group consisting of 1018 ISS, Aluminum Salt, Amplivax, AS15, BCG, CP-870,893, CpG ODN, CpG7909, SIA, dSLIM, GM-CSF, IC30, IC31, Lmiquimod, Imufact IMP321, IS Patch, Iscomatrix, Juvlmmune, Lipovac, MF59, Monophosphoryl Lipid A, Montanide IMS 1312, Montanide ISA 206, Montanide ISA 50V, Montanide ISA-51, OK-432, OM-174, OM-197-MP-EC, ONTAK, PepTel Vector System, PLG Microparticles, Resiquimod, SRL172, Virosomes and other Virus-like Particles, YF-17DBCG, Aquila's QS21 stimulon, Ribi's Detox. Quil, Superfos, Freund's, GM-CSF, Cholera Toxins, Immunological Adjuvants, MF59 and Cytokines.

16. The vaccine composition according to claim 14, wherein the immune checkpoint inhibitor is at least one selected from the group consisting of a programmed cell death-1 (PD-1) antagonist, a programmed cell death-ligand 1 (PD-L1) antagonist, programmed cell death-ligand 2 (PD-L2) antagonist, cluster of differentiation 27 (CD27) antagonist, cluster of differentiation 28 (CD28) antagonist, cluster of differentiation 70 (CD70) antagonist, cluster of differentiation 80, also known as B7-1 (CD80) antagonist, cluster of differentiation 86, also known as B7-2 (CD86) antagonist, cluster of differentiation 137 (CD137) antagonist, cluster of differentiation 276 (CD276) antagonist, killer-cell immunoglobulin-like receptors (KIRs) antagonist, lymphocyte-activation gene 3 (LAG3) antagonist, tumor necrosis factor receptor superfamily, member 4, also known as CD134 (TNFRSF4) antagonist, glucocorticoid-induced TNFR-related protein (GITR) antagonist, glucocorticoid-induced TNFR- related protein ligand (GITRL) antagonist, 4-1BB ligand (4-1BBL) antagonist, cytolytic T lymphocyte associated antigen-4 (CTLA-4) antagonist, Adenosine A2A receptor (A2AR) antagonist, V-set domain-containing T-cell activation inhibitor 1 (VTCN1) antagonist, B- and T-lymphocyte attenuator (BTLA) antagonist, indoleamine 2,3-dioxygenase (IDO) antagonist, T-cell Immunoglobulin domain and Mucindomain (3TIM-3) antagonist, V-domain Ig suppressor of T cell activation (VISTA) antagonists and killer cell lectin-like receptor subfamilyA (KLRA) antagonists.

17. A pharmaceutical composition for preventing or treating cancer comprising at least one selected from the group consisting of the following (i) to (iv):
(i) the peptide of claim 1,
(ii) a polynucleotide encoding (i) the peptide;
(iii) a vector comprising (ii) the polynucleotide, and
(iv) a host cell transformed by (iii) the vector.

18. Use of the at least one selected from the group consisting of the following (i) to (iv) for preparation of an agent for the treatment of cancer:
(i) the peptide of claim 1,
(ii) a polynucleotide encoding (i) the peptide;
(iii) a vector comprising (ii) the polynucleotide, and
(iv) a host cell transformed by (iii) the vector.

19. A method for treating cancer comprising administering to a subject in need thereof an effective amount of a composition comprising at least one selected from the group consisting of the following (i) to (iv):
(i) the peptide of claim 1,
(ii) a polynucleotide encoding (i) the peptide;
(iii) a vector comprising (ii) the polynucleotide, and
(iv) a host cell transformed by (iii) the vector.
